(19)

**Europäisches Patentamt**

**European Patent Office**

**Office européen des brevets**

(11) **EP 4 763 976 A1**

(12) **EUROPEAN PATENT APPLICATION**

(43) Date of publication:
24.06.2026 Bulletin 2026/26

(21) Application number: 24223011.8

(22) Date of filing: 23.12.2024

(51) International Patent Classification (IPC):
*C12N 5/077* (2010.01)    *A61K 35/34* (2015.01)

(52) Cooperative Patent Classification (CPC):
**A61K 35/34; C12N 5/0658;** C12N 2513/00;
C12N 2531/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: Innovacell GmbH
6020 Innsbruck (AT)

(72) Inventors:
• **Potocki, Hannah**
6020 Innsbruck (AT)
• **Hutz, Jasmina**
6020 Innsbruck (AT)
• **Torggler-Kreidl, Raffaela**
6020 Innsbruck (AT)
• **Erharter, Anita**
6020 Innsbruck (AT)

(74) Representative: **Drexl, Janna**
**Dehmel & Bettenhausen**
**Patentanwälte PartmbB**
**Herzogspitalstraße 11**
**80331 München (DE)**

(54) **METHODS FOR OBTAINING AN EXPANDED POPULATION OF SKELETAL MUSCLE DERIVED CELLS**

(57)    The present invention relates to methods for obtaining an expanded population of skeletal muscle derived cells (SMDC). The present invention also refers to an expanded population of SMDCs obtained by the method according to the present invention and to an expanded population of SMDCs. Further, the present invention refers to an expanded population of SMDCs for use in a method for treatment of the human or animal body by surgery or therapy or for use as a pharmaceutical composition. In addition, the present invention refers to an expanded population of SMDCs for use in a method of preventing and/or treating neuromyopathies, myopathies and/or muscle dysfunctions such as a urinary and/or an anal incontinence.

EP 4 763 976 A1

**EP 4 763 976 A1**

**Description**

The present invention relates to methods for obtaining an expanded population of skeletal muscle derived cells (SMDC). The present invention also refers to an expanded population of SMDCs obtained by the method according to the present invention and to an expanded population of SMDCs. Further, the present invention refers to an expanded population of SMDCs for use in a method for treatment of the human or animal body by surgery or therapy or for use as a pharmaceutical composition. In addition, the present invention refers to an expanded population of SMDCs for use in a method of preventing and/or treating neuromyopathies, myopathies and/or muscle dysfunctions such as a urinary and/or an anal incontinence.

Skeletal muscle derived cells are the drug substance of investigational drug products as e.g. outlined in WO 2019/115790 and WO 2023/012334 for the treatment of faecal- and urinary incontinence, respectively. Briefly, SMDCs are isolated from skeletal muscle biopsy and cultivated to reach the therapeutically effective cell number. They mainly consist of the proposed active compound of myogenic cells (CD56$^+$ cells), progenitor cells with a crucial role for muscle regeneration and formation of multinucleated myotubes. On the other hand, non-myogenic cells (cells that are isolated from muscle biopsy but are CD56$^-$) are unable to form myotubes *in vitro* and are therefore considered as product related impurity. Data obtained from research and (pre-)clinical studies showed that in CD56 mixed cultures, the proportion of CD56$^+$ myogenic cells decreased during the expansion phase while CD56$^-$ non-myogenic cells eventually outgrew the myogenic population. The final product should, however, consist of 80% $\pm$ 20% CD56$^+$ SMDCs to ensure sufficient regenerative potential and efficient myotube formation following transplantation. Thus, it is of utmost importance to provide optimal culture conditions which favour growth of CD56$^+$ myogenic cells throughout expansion to obtain a high-quality myogenic product for final formulation of myogenic cells (CD56$^+$ cells) for medical applications.

Moreover, a higher degree of automation for manufacturing is desired, and reduction in terms of i) workload within the cleanroom facility and the necessary process steps for manufacturing, ii) incubator space per batch to increase production capacity, as well as iii) plastic ware and media volume for higher cost efficiency are required. Briefly, the current manufacturing process involves manual processing of skeletal muscle biopsy and isolation of the myogenic population, which is followed by sequential expansion by two-dimensional (2D) cell culture, including several manual splitting steps. As automation of the two-dimensional (2D) expansion culture setup is not feasible, a promising and state-of-the-art solution is the transition to three-dimensional (3D) culture of myogenic cells (CD56$^+$ cells) using microcarriers. Microcarriers are small spheres with diameters of 100 to 300 $\mu$m, consisting of matrices that allow growth of adherent cells and thereby facilitate their cultivation in bioreactors. Bioreactor systems considerably reduce the space needed for cultivation, the amount of plastic ware and culture medium, as well as manual handling steps. Furthermore, several semi-automated systems are readily available by different manufacturers.

Methods for preparing a mass culture of muscle precursor cells (MPCS) are for example described in WO 2024/115761 A1. However, the muscle precursor cells (MPCS) obtained in WO 2024/115761 A1 comprise a maximum of 15 % CD56 positive cells.

Thus, new methods for enriching and expanding myogenic cells comprising a minimum of 60% myogenic, CD56 positive cells are needed.

The object underlying the present invention is, therefore, the provision of new methods for expanding myogenic cells comprising a minimum of 60% myogenic, CD56 positive cells. Said method should be faster and more cost effective than methods known in the art, e.g. by needing less material for cell culture such as cell growth medium. In particular, it should be partly or completely automatable so that less man power is needed. Another object underlying the present invention is the provision of a method in which myogenic CD56 expressing cells are not depleted over CD56 non-expressing cells, in particular if the expansion is started with a mixture comprising a significant amount (e.g. more than 10%) of CD56 non-expressing cells. In fact, one of the objects of the present invention is the provision of a method for expanding CD56 expressing cells, wherein the ratio of CD56 expressing cells to CD56 non-expressing cells is stable or even increased during expansion. Thus, the object is to provide a method in which the proportion of CD56 expressing cells in the total cell number is stable or even increased during expansion. Said method should allow the provision of an effective amount of autologous cells, preferably more than 40 or even more than 80 million cells, which are able to be used for medical applications, preferably by local injection in a subject in need thereof. Preferably, said method allows the provision of so many cells that it is not only sufficient for one medical application but for more than one such as 2 or 3 applications or repeated injections. Said cells should be myogenic and able to fuse into myofibers after local injection in said subject. Thus, it is also an object of the present invention to provide a process for expanding myogenic cells which does not result in premature fusion. Premature fusion is an unwanted side effect resulting from dense myogenic progenitor growth, which can trigger spontaneous fusion events even in the absence of differentiation medium. Another object of the present invention is the provision of a safe method for expanding said myogenic cells, wherein the obtained, expanded cells meet high and constant quality standards. Said high and constant quality standards are highly important for minimizing any potential risks for the subject in need thereof. As safety concerns may for example arise if cells for cell therapy are obtained by cell sorting methods such as MACS and FACS (due to possible damaging of the cells by applying high voltage laser intensities in FACS and concerns about magnetic antibody retention using MACS) it is another object of the present invention to provide methods that do not require cell sorting such as FACS and MACS. Another object of the present invention is the provision of a robust method for providing a high-quality cell product which can be used for all kinds of skeletal muscle derived cells obtained from skeletal muscle tissues. This means that the high-quality cell product can both be obtained from high quality samples of skeletal muscle tissue comprising a high amount of CD56 expressing cells, but also from lower quality samples of skeletal muscle tissue comprising a lower amount of CD56 expressing cells, e.g. due to the age of the subject from which the skeletal muscle tissue was obtained.

A further object of the present invention is the provision of a population comprising optimized myogenic, CD56 positive cells having increased abilities to survive and to fuse into myofibers after local injection in a subject in need thereof. Preferably, said optimized myogenic CD56 positive cells show increased cell adhesion and differentiation which enhances myotube formation, in particular after they have been administered or injected in or adjacent to muscle tissue.

These objects are solved by the subject matter defined in the claims.

The following figures form part of the present specification and are included to further demonstrate certain aspects of the present invention. The invention may be better understood by reference to one or more of these drawings in combination with the detailed description of specific embodiments presented herein.

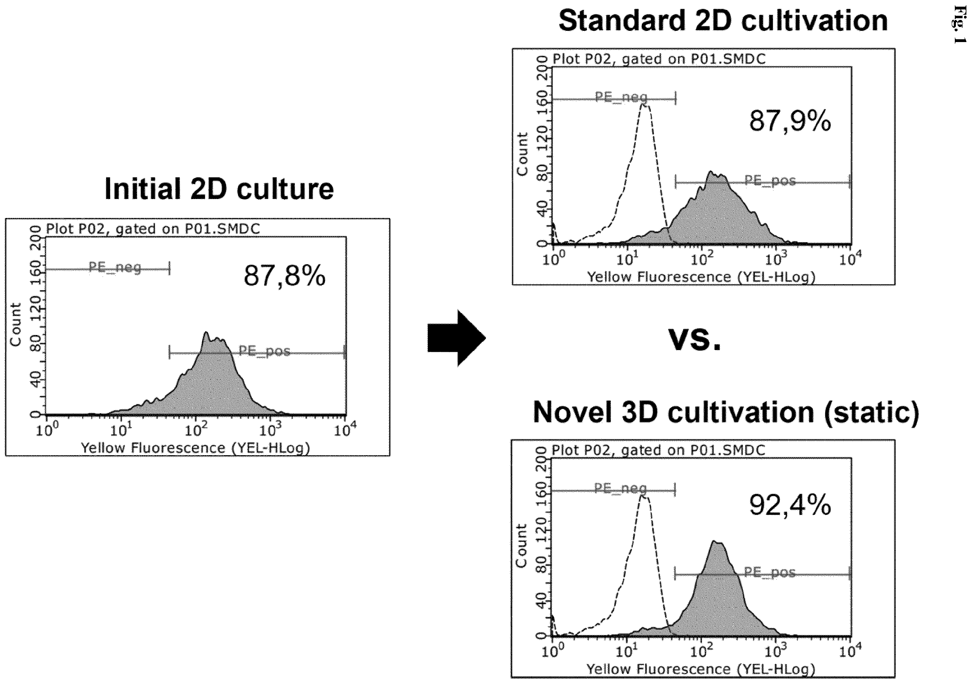

Figure 1 shows CD56 expression of SMDCs after cultivation on *Hillex II* microcarriers under static conditions. Stable to slightly increased CD56 expression was observed, suggesting a beneficial effect of Hillex II microcarriers in 3D culture for myogenic cells.

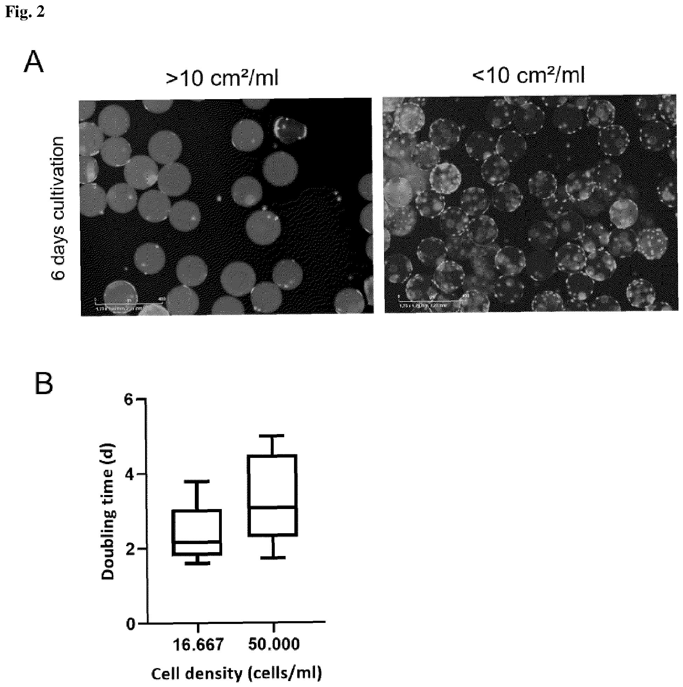

Figure 2 shows impact of microcarrier density on SMDCs growth. (A) Syto24 staining of cell nuclei on Hillex II microcarriers. At a microcarrier density of >10 $cm^2$/ml (17.5 $cm^2$/ml shown in image), cells were initially able to attach without further growth, in contrast to microcarrier densities <10 $cm^2$/ml (4.8 $cm^2$/ml shown in image) where cells were proliferative. (B) Comparison of cell growth at different seeding densities revealed increased doubling times at higher seeding densities, while doubling times at lower seeding densities were comparable to 2D culture of approximately two days.

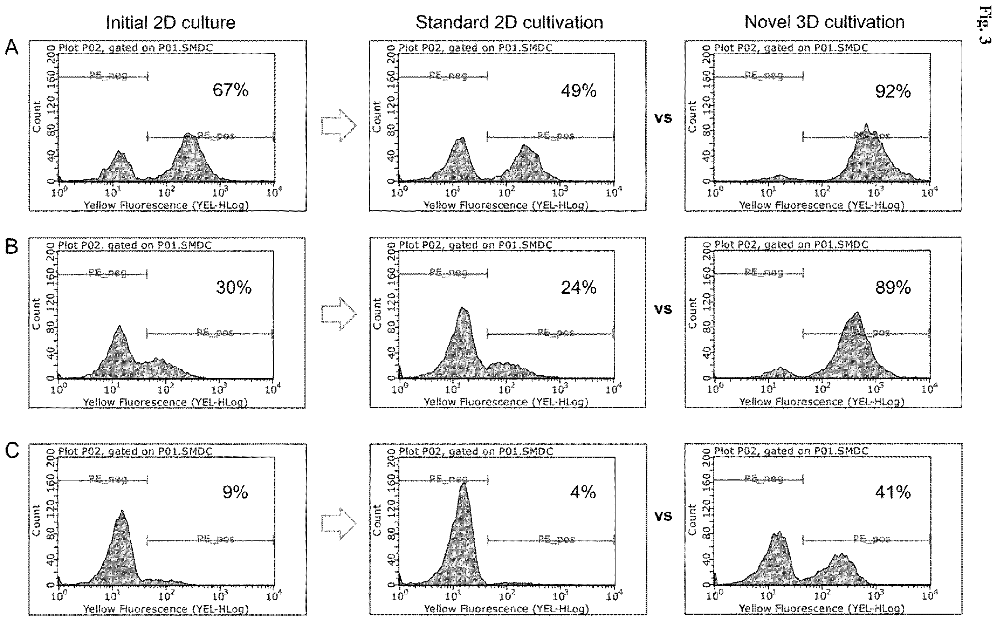

Figure 3 shows enhanced CD56 expression in SMDCs following cultivation on Hillex II microcarriers in spinner flasks. After seven to nine days of cultivation with the microcarrier / spinner flask system, notable increases from (A) 67% to 92%, (B) 30% to 89%, and (C) 9% to 41% CD56+ SMDCs was observed. In contrast, parallel cultivation of the same cell batch by standard 2D cultivation resulted in a decrease of CD56+ cells.

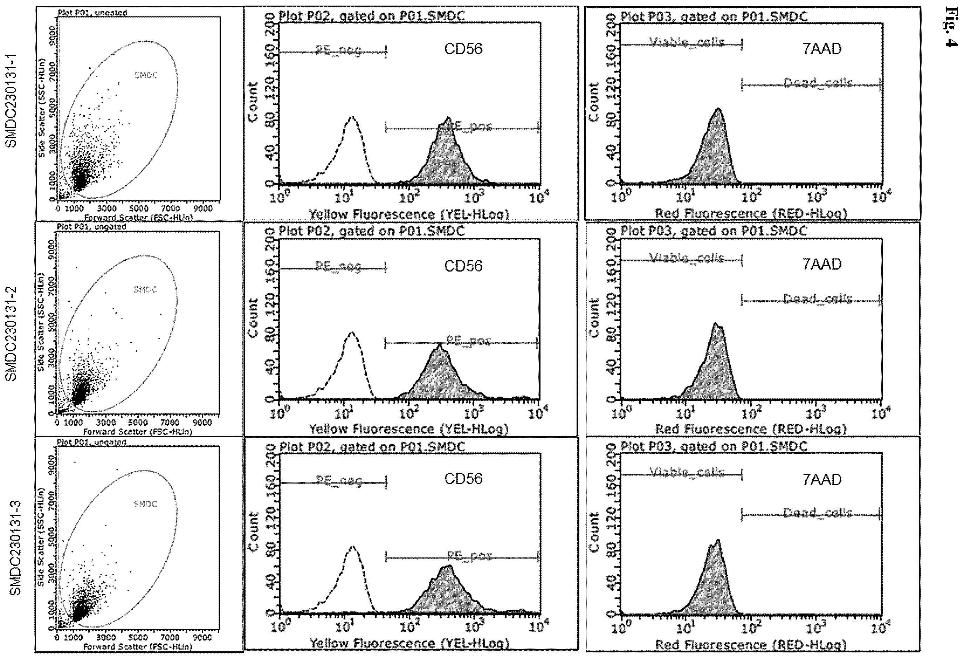

Figure 4 shows flow cytometry analysis of CD56 expression following 3D expansion of SMDC. High expression levels of >97% CD56+ for SMDCs from all three biopsies after real-scale expansion to >50 million cells. Additionally, cells were viable as measured by 7AAD.

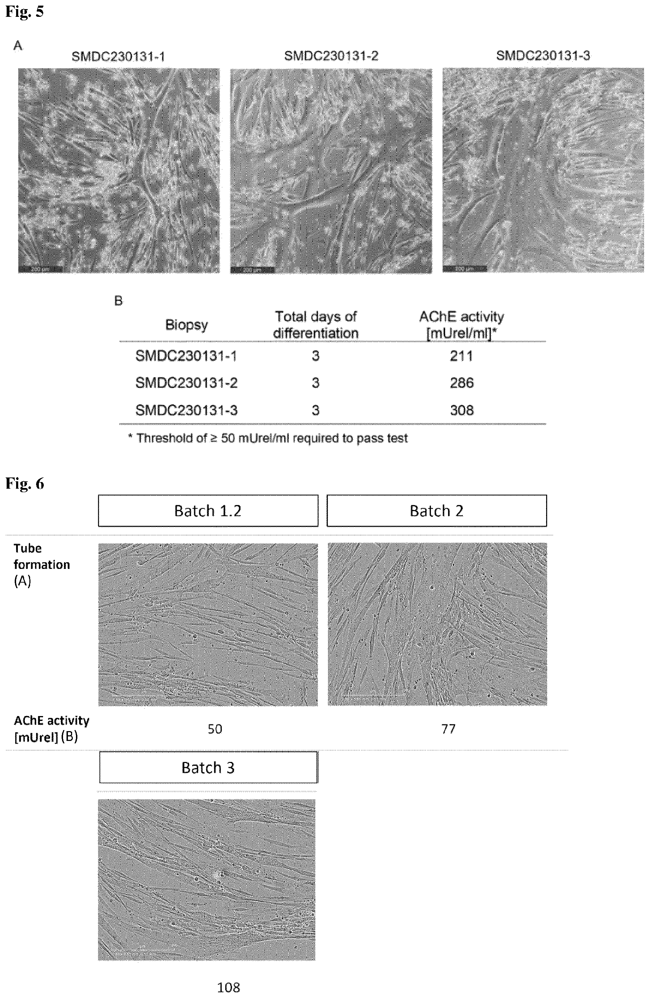

Figure 5 shows fusion capability and AChE activity in 3D cultured SMDC. SMDCs obtained following real-scale expansion in 3D microcarrier / spinner flask system (A) demonstrated robust fusion and myotube formation capability, and (B) passed AChE potency assay. Scale bars in (A) indicate 200 $\mu$m. Images were acquired with Leica DMi1.

Figure 6 shows successful fusion of bioreactor-derived SMDCs to myotubes and pass of AChE potency test. A) Formation of multi-nucleated tubes upon differentiation. B) Relative Acetylcholine esterase activity was passed (threshold $\geq$ 50 mUrel / 200.000 cells).

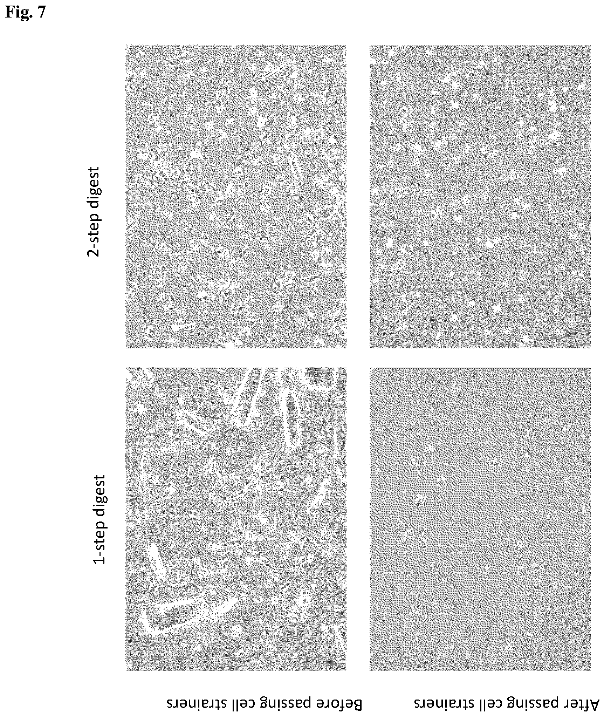

Figure 7 shows comparison of obtained cells after a one step digest performed for 20 hours at 37°C with collagenase and a two-step digest, wherein the first digestion step is performed for 20 hours at 37°C with collagenase followed by a second digestion step performed for 10 min at 37°C with Trypsin. The obtained cells are shown before and after they have passed a series of cell strainers (with 100, 40 and 15 $\mu$m mesh size).

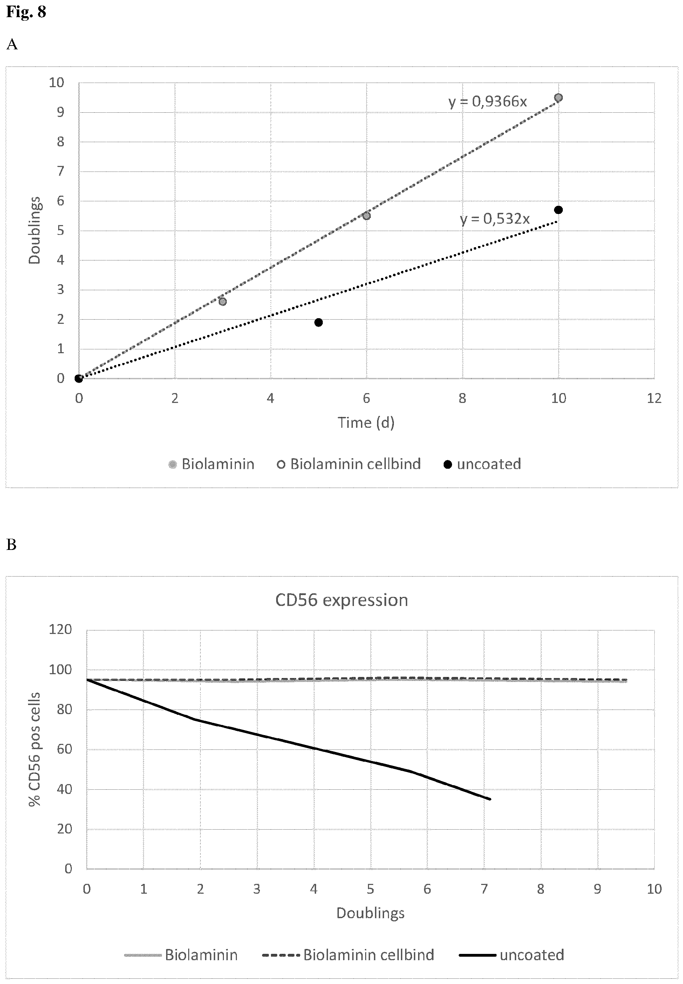

Figure 8 (A) shows the growth rates (doublings per time) of cells on Biolaminin-coated flasks and uncoated flasks. Biolaminin and Biolaminin CellBIND displayed identical growth rate. Figure 8 (B) shows percentage of CD56 positive cells in relation to the number of doublings on uncoated flasks or flasks coated with either Biolaminin or Biolaminin CellBIND.

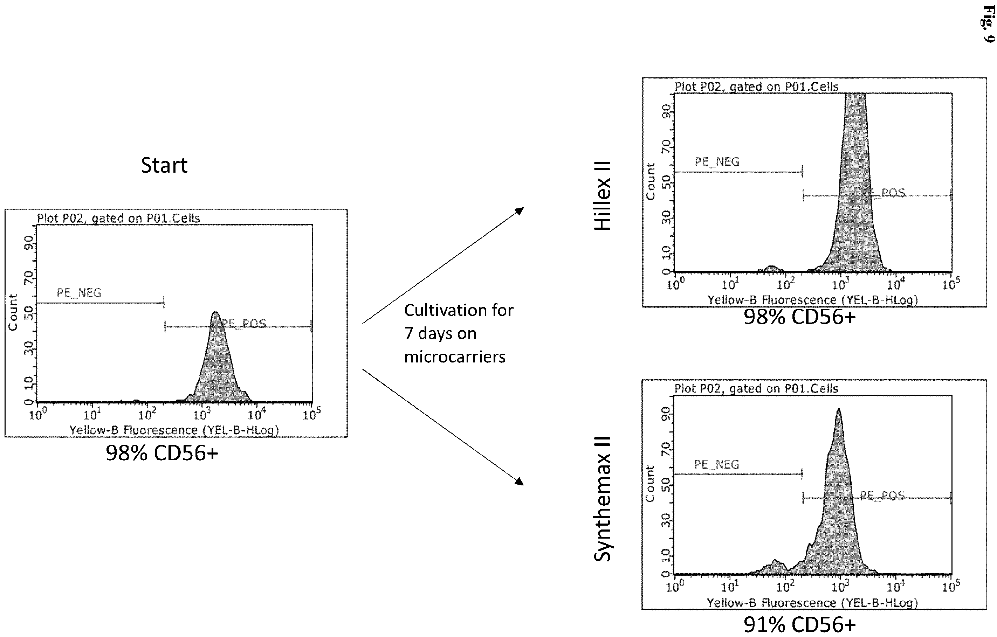

Figure 9 shows flow cytometry analysis of CD56 expression of starting culture and after cultivation for 7 days on microcarriers on either Hillex II or Synthemax II. The proportion of CD56 expressing SMDCs stayed basically constant in both systems (on Hillex II microcarriers +0%; on Synthemax II microcarriers -7%) and above 90%.

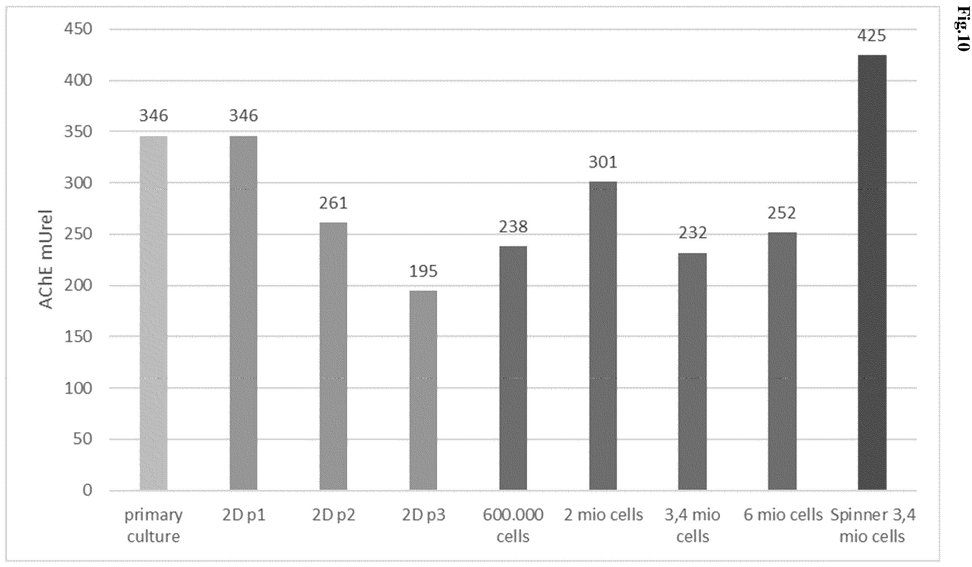

Figure 10 shows the results of the AChE potency assay of cells obtained after primary culture, after passage 1 in 2D culture, after passage 2 in 2D culture, after passage 3 in 2D culture, after culture with Hillex II microcarriers in DASbox bioreactor starting with distinct seeding densities (600.000 cells, 2 mio cells, 3.4 mio cells and 6 mio cells) and after culture in a spinner flask and seeding density of 3.4 mio cells.

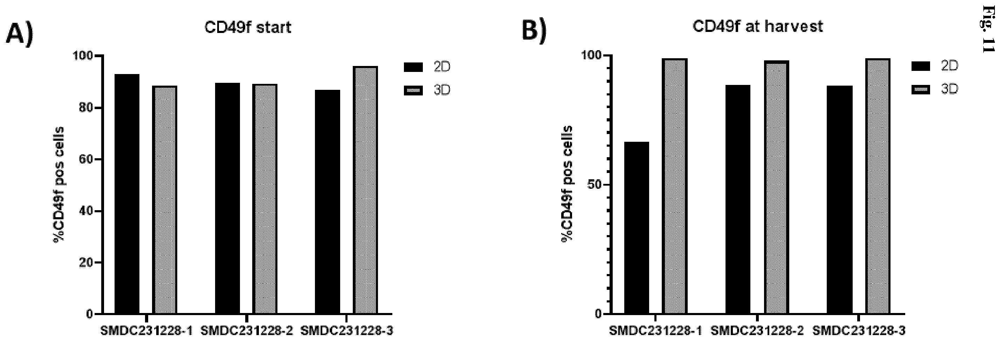

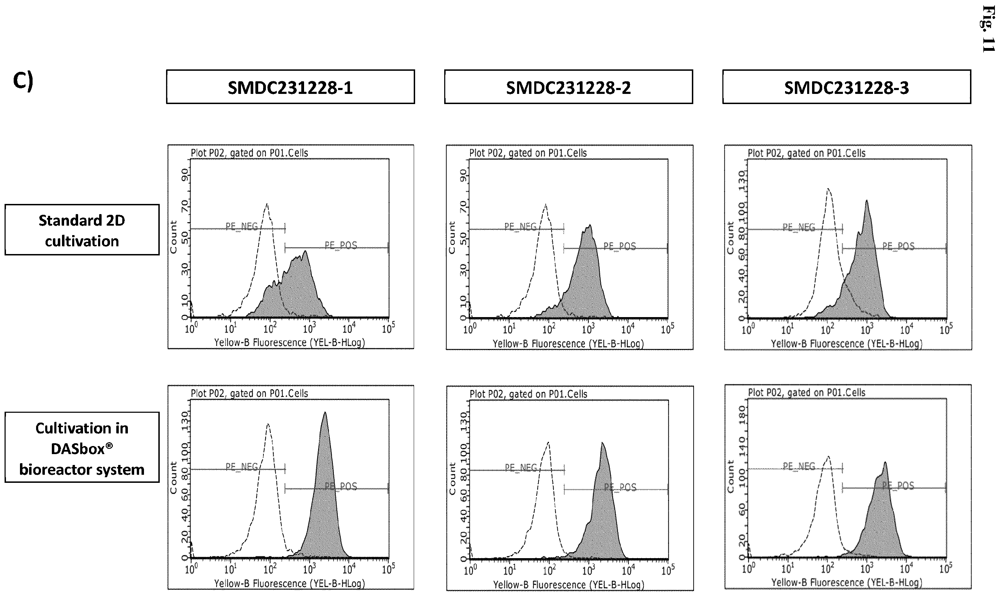

Figure 11 shows CD49f expression analysis by flow cytometry. A) The percentage of CD49f+ cells after primary culture was comparable between cells before cultivation in the DASbox® bioreactor system (3D) and those after the second passage in the standard 2D procedure. B) However, following cultivation, the percentage of CD49f+ cells was significantly increased (by 9-32%) after cultivation in the DASbox® bioreactor system compared to 2D cultivation. C) Flow cytometry analysis at harvest reveals the absence of a CD49f- population following 3D cultivation, in contrast to the presence of a (small) CD49f- fraction observed after 2D cultivation.

The use of the word "a" or "an" when used in conjunction with the term "comprising" in the claims and/or the specification may mean "one," but it is also consistent with the meaning of "one or more," "at least one," and "one or more than one."

The term "about" means that the value stated, plus or minus 5% of the stated value, or the standard error for measurements of the given value, are contemplated.

The term "comprising" as used herein shall not be construed as being limited to the meaning "consisting of" (i.e. excluding the presence of additional other matter). Rather, "comprising" implies that optionally additional matter may be present. The term "comprising" encompasses as particularly envisioned embodiments falling within its scope "consisting of" (i.e. excluding the presence of additional other matter) and "comprising but not consisting of" (i.e. requiring the presence of additional other matter), with the former being more preferred.

The terms "CD56$^+$", "CD56 positive" or "CD56 expressing cell" as used herein refers to a cell expressing the cell marker CD56, preferably on its surface. The terms "CD56$^+$" or "CD56 positive" can also be used for a cell population comprising different cell types, if preferably at least 50, 60, 70, 80, 90, 95, 98 or 99 percent of the cell population express the cell marker CD56.

The term "CD56$^-$", "CD56 negative" or "CD56 non-expressing cell" as used herein refers to a cell not expressing the cell marker CD56. The terms "CD56$^-$", "CD56 negative" or "CD56 non-expressing cells" can also be used for a cell population comprising different cell types, if preferably less than 50, 40, 30, 20, 10, 5, 4, 3, 2, 1 or 0 percent of the cell population express the cell marker CD56.

In accordance with the definitions of the terms "CD56$^+$", "CD56 positive", "CD56 expressing cells", "CD56$^-$", "CD56 negative" or "CD56 non-expressing cell" the marker CD56 may be replaced by any other cell marker as used herein such as CD105, CD90, CD34, CD49f , alpha-7 (ITGa7), Myogenic factor 5 (Myf5), Desmin, SSEA4, myosin heavy chain (MHC) or smooth muscle actin (SMA). Thus, referring to a positive expression of any of those markers means that least 50, 60, 70, 80, 90, 95, 98 or 99 percent of the cell population express said cell marker, preferably on their surface, wherein referring to a negative expression of any of those markers means that less than 50, 40, 30, 20, 10, 5, 4, 3, 2, 1 or 0 percent of the cell population express said cell marker, unless otherwise specified.

The term "myogenic potential" refers preferably to the possibility of a cell, cells, or a cell population to develop, regenerate and/or augment muscle tissue. Myogenic potential as used herein may further relate to the potential of a cell, cells or a cell population to differentiate in vitro to multinucleated myotubes and/or express enzymatically active Acetylcholinesterase.

The term "myogenic differentiation potential" or in short "myogenic potential" as used herein preferably refers to the ability of a cell to express detectable amounts of proteins known to be expressed by myogenic cells in vivo, such as but not limited to one or more of the following markers of Desmin, CD56, Myf5, ITGA7 and Myosin. Tests for expression of such markers in in vitro cultivated cells are well known to the skilled person in the art. Such tests preferably involve flow cytometric and/or other immunocytochemical assays including fluorescent immunostainings and western blotting. Alternatively, preferred is the use of the term myogenic differentiation potential referring to the ability of a cell (e.g. myogenic progenitor cell) to form multinucleated myotubes. Such myotubes are known to the skilled person in the art as muscle cells comprising preferably at least 3 separate nuclei. Formation of such myotubes by originally single nucleated cells in e.g. a differentiation medium may thus be used as a test for myogenic differentiation potential.

The term "fusion competent" or "skeletal-myogenic" as used herein refers to cells able to fuse to multinucleated myotubes with at least 50, 60, 70, 80, 90 or 100 percent of nuclei within multinucleated myotubes following cultivation in skeletal muscle differentiation media for 5-7 days.

The term "myotube" as used herein, preferably refers to a muscle cell comprising at least 3 separate nuclei. Preferably, such myotube is formed by fusion of single nucleated cell (e.g. myogenic progenitor cells).

The term "AChE positive" or "AChE+" as used herein preferably refers to a cell or cell population showing an (i.e. a positive) acetylcholinesterase enzymatic (AChE) activity. In particular, such an AChE activity is positive if $2*10^5$ cells show an AChE activity of about 20 mU$_{rel}$ to about 1000 mU$_{rel}$, more preferably about 30 mU$_{rel}$ to about 800 mU$_{rel}$, and even more

EP 4 763 976 A1

preferable about 50 mU$_{rel}$ to about 700 mU$_{rel}$. Preferably, said $2*10^5$ cells have been cultivated in skeletal muscle differentiation medium as e.g. described in the examples herein. The unit "mU$_{rel}$" refers to a relative "mU/ml" of $2*10^5$ cells measured 60 minutes after addition of Acetylthiocholine iodide (ATI) and 5,5'-Dithiobis(2-nitrobenzoic acid) (DTNB), in respect of a straight-line equation obtained by a dilution series of an AChE stock solution ranging from 4-500 mU/ml under the same conditions except for that the OD of the dilutions of the stock solution is already measured 6-8 min, preferably 6, 7, or 8 min, after addition of ATI and DTNB. The term "mU$_{rel}$" may also refer to a relative "mU/ml" of $2*10^5$ cells as measured and calculated in Example 7.

The term "AChE negative" or "AChE-" as used herein preferably refers to a cell or cell population having no (i.e. a negative) AChE activity. In particular, such an AChE activity is negative if $2*10^5$ cells show an AChE activity of about 0 mU$_{rel}$ to about 19 mU$_{rel}$, more preferably of about 0 mU$_{rel}$ to about 29 mU$_{rel}$, 0 mU$_{rel}$ to about 39 mU$_{rel}$ or 0 mU$_{rel}$ to about 49 mU$_{rel}$, wherein the cells have preferably been cultivated in skeletal muscle differentiation medium as e.g. described in Example 7 herein.

The terms "cell growth medium", "culture medium" or "growth medium" as used herein refers to any medium suitable for the incubation of mammalian cells such as SMDCs, in particular human SMDCs, which allows the attachment of said mammalian or human cells on a suitable surface and/or the expansion of said cells.

The term "microcarrier" as used herein refers in general to a support matrix used in cell culture for increasing the growth surface area in cell culture. Usually, microcarriers are small spherical particles that allow cells to adhere on their surface and that allow adherent cells to grow on their surfaces. Microcarriers are preferably composed of polysaccharides such as dextran, glass, plastic material such as polystyrene and may be uncoated or coated with components such as collagen or gelatin.

The term "microporous" refers to a solid material, preferably a microcarrier, that has very small openings that allow air and moisture to pass through, while preventing larger particles from getting in.

The term "skeletal muscle derived cells" or "SMDCs" refers to cells or a cell population obtained from skeletal muscle tissue. Said SMDCs comprise amongst others cells having a myogenic potential which means that they are fusion competent, multinucleated fusion competent or skeletal-myogenic. Said fusion competent, multinucleated fusion competent or skeletal-myogenic cells are preferably CD56 positive. SMDCs can be primary cells and/or in vitro cultured cells. Said SMDCs may further comprises cells not having a myogenic potential. Such cells are called "non-myogenic cells" or "skeletal muscle derived non-myogenic cells" herein and are preferably CD56 negative and/or desmin negative.

The term "2D culture", as used herein, refers to a cell culture performed on a flat surface, typically a petri dish or a flask. In contrast, the term "3D culture", as used herein, refers to a cell culture on three dimensional microcarriers, typically having a spherical form. Said 3D culture is preferably performed in a bioreactor.

The term "penetration," as used herein, refers to a process of introducing an injection device, for instance a needle into a body tissue without affecting the injection process yet. The term "injection", as used herein, refers to the expulsion of an injection solution comprising above mentioned cells out of an injection device into a specific site within the human body, in particular into or adjacent to muscle-tissue providing for anal continence. The injection process can be, but is not limited to, static, *i.e.,* the injection device remains at the position reached. Alternatively, the injection process is dynamic. For instance, in some embodiments of the present invention the injection occurs simultaneously with the retraction of the injection device from the site of injection. The term "injection site", as used herein, refers to a site within the human body, such as close to or being muscle-tissue providing for anal continence, at which the injection process is initiated. The injection site needs not to be identical with the site where the injection process ends.

The terms "faeces incontinence" or "anal incontinence" as used herein, refer to the undesired loss of liquid or formed faeces through the anus. Said terms comprise passive incontinence, imperative defecation and imperative urgency. The term "passive incontinence", as used herein, refers to a lack of sensory recognition of loss of faeces. This comprises low anal base line pressure values and a lacking sensoric ability of the anal and rectal mucosa. "Imperative defecation" or "imperative urgency", as used herein, refers to the lacking ability of a person to delay defecation for more than five minutes. Such a patient has to go to the toilette immediately.

In accordance with the present invention, methods for obtaining CD56 expressing skeletal muscle derived cells (CD56$^+$ SMDCs) are provided. Preferably, said methods are methods for obtaining and expanding CD56 expressing SMDCs. Said expansion preferably comprises the propagation of CD56 expressing SMDCs which in turn results in the increase of the cell number of CD56 expressing SMDCs. Moreover, said methods may result in the enrichment of CD56 expressing SMDCs, in particular over SMDCs not expressing the cell marker CD56. CD56$^+$ SMDCs are preferably a population of SMDCs comprising at least 60% CD56 expressing cells.

Thus, a first subject-matter of the present invention is directed to a method for obtaining an expanded population of skeletal muscle derived cells (SMDCs) comprising at least 60% CD56 expressing cells. Preferably, at least 70% of the cells of said cell population express the cell marker CD56, more preferably at least 80, 85%, 90%, 93%, 95%, 97%, 98%, 99% or at least 100%. The CD56 expressing SMDCs of the expanded population of SMDCs according to the present invention may be referred as "CD56$^+$ SMDCs" or "SMDCs expressing CD56" herein. Said CD56$^+$ SMDCs are preferably myogenic, i.e. they have myogenic potential, more preferably myogenic differentiation potential. Preferably, said CD56$^+$

SMDCs are committed to the myogenic lineage, more preferably to the skeletal myogenic lineage. This means that the CD56$^+$ SMDCs are dedicated to develop into muscle cells, more preferably skeletal muscle cells.

The method of the present invention comprises the following steps:

(a) providing a suspension in a container comprising

(i) skeletal muscle derived cells (SMDCs) obtained from skeletal muscle tissue,
(ii) microcarriers, and
(iii) culture medium,

(b) cultivating the suspension under

mixing in a given volume, and
changing the medium several times, and
conditions allowing the expansion of said SMDCs,

thereby obtaining an expanded population of SMDCs comprising CD56 expressing SMDCs,
(c) and optionally harvesting the population of expanded SMDCs attached to the microcarriers.

The cultivation and/or expansion of CD56 expressing SMDCs often results in a depletion of CD56 expressing SMDC, in particular if a mixture of CD56$^+$ and CD56$^-$ SMDCs are used as starting material. This means that during cultivation and/or expansion the ratio of CD56 expressing SMDCs (CD56$^+$ SMDCs) to SMDCs not expressing the cell marker CD56 (CD56$^-$SMDCs) is shifting in favour of the CD56$^-$ SMDCs. This also means that during cultivation and/or expansion the proportion of CD56 expressing SMDCs in the total cell number decreases while the proportion of CD56$^-$ SMDCs increases. The inventors have surprisingly found that conducting the method of the present invention advantageously allows stabilizing or even enriching the proportion of CD56 expressing SMDCs in the total cell number and over CD56 non-expressing SMDCs cells. If the starting material comprises already a high amount of CD56$^+$ SMDCs (e.g. more than 80%, 90% or 95% CD56$^+$ cells) said depletion effect seems to be not so pronounced or does not occur at all. But also under these circumstances conducting the method of the present invention advantageously allows stabilizing or even further enriching the proportion of CD56 expressing SMDCs in the total cell number. Moreover, the present invention allows obtaining and expanding said CD56 expressing SMDCs, wherein the obtained cell population exhibits high myogenic capacity. These obtained CD56$^+$ SMDCs are highly pure for myogenic markers such as CD56 and desmin, indicating increased myogenic potential as assayed for example by a suitable potency assay. Moreover, the method of the present invention allows the provision of SMDCs in an amount sufficient for use in medical applications such as urinary and/or faecal incontinence. Thus, the expanded population of SMDCs obtained by the present invention demonstrate high purity and viability. Therefore, it can be expected that it has high clinical efficacy in supporting the neuro-muscular connection as well as regenerating muscle weakness especially in conditions as urinary and/or faecal incontinence.

In particular in standard 2D cultivation, mixed SMDCs cultures (i.e. SMDCs culture comprising both CD56$^+$ and CD56$^-$ SMDCs) are at risk of being overgrown by the CD56$^-$ population during the expansion process. This effect increases with the number of passages and/or the duration of the cultivation. It was surprisingly found that the opposite effect was observed in 3D cultivation according to the method of the present invention: 3D cultivation of CD56$^+$ and CD56$^-$ SMDCs resulted in a stabilization or even enrichment of the CD56$^+$ population over the CD56$^-$population. Said observation cannot be explained by impaired growth of CD56$^-$ cells on the microcarriers. Rather, in the mixed population of SMDCs the CD56$^+$ population seems to gain a growth advantage over the present CD56$^-$ cells, presumably caused by the three dimensional culture setup using microcarriers, the specific conditions for 3D cultivation as described herein and/or a combination of both, which ultimately results in decrease of the CD56$^-$ population. Thus, the expansion according the present invention is preferably performed under conditions which are at least as favorable for CD56 expressing SMDCs as for CD56 non-expressing SMDCs. Preferably, the expansion is performed under conditions which are more favorable for CD56 expressing SMDCs than for CD56 non-expressing cells which results in an enrichment of CD56 expressing cells and a depletion of CD56 non-expressing cells.

CD56 also known as neural cell adhesion molecule (NCAM) is a myogenic commitment marker expressed in skeletal muscle myoblasts in vitro and smooth muscle tissue in vivo. CD56 is present in fusion competent desmin$^+$ SMDC. CD56 in particular has been demonstrated to mark a SMDCs population that is able to form multinucleated myotubes and express higher enzymatic acetylcholinesterase (AChE) activity than CD56 negative SMDCs (Thurner et al., 2018. PLoS ONE 13(3): e0194561). High AChE activity of SMDCs used to treat fecal incontinent patients in fact has been linked to high treatment success in terms of reduction of fecal incontinence symptoms (Thurner et al., 2018. PLoS ONE 13(3): e0194561). Thus, SMDCs highly pure for CD56 and thus high in AChE activity are desired for successful treatment of patients with neuromyopathies and/or myopathies such as urinary or fecal incontinence. The present invention provides a method to provide an expanded population of SMDCs highly pure for CD56 expressing cells and with high AChE activity.

Thus, in a preferred embodiment of the present invention, the expanded population of SMDCs obtained in step (b) comprise preferably at least 60%, 70% or most preferably at least 80% CD56 expressing cells. In a further preferred embodiment, the proportion of CD56 expressing SMDCs in the total cell number of SMDCs increases during expansion in step (b) or decreases at most by 10%.

Preferably, the suspension in step (a) comprises about 0.5 to about 10 million cells, or about 1 to about 7 million cells, about 2 to about 6 million cells, about 3 to about 5 million cells, or about 2 to about 5 million cells.

Preferably, the suspension in step (a) comprises an amount of microcarriers providing a growth surface area for the cells of about 500 to about 2000 $cm^2$, about 600 to about 1900 $cm^2$, about 700 to about 1800 $cm^2$, about 800 to about 1700 $cm^2$, about 800 to about 1600 $cm^2$, about 900 to about 1500 $cm^2$, about 1000 to about 1400 $cm^2$, about 1100 to about 1300 $cm^2$ or about 1200 $cm^2$.

Preferably, the suspension in step (a) comprises seeding densities of about 500 to about 17,000 cells/$cm^2$ of about 1000 to about 7000 cells per $cm^2$ of growth surface area provided by the microcarriers, more preferably about 1500 to about 6000 cells/$cm^2$ or about 1700 to about 5000 cells/$cm^2$, or about 1800 to about 4500 cells/$cm^2$, or about 1900 to about 4000 cells/$cm^2$, or about 2000 to about 4500 cells/$cm^2$, or about 1900 to about 3500 cells/$cm^2$, or about 1800 to about 3500 cells/$cm^2$ or about 2000 to about 3500 cells/$cm^2$, or about 1700 to about 4000 cells/$cm^2$.

The volume of the suspension in step (a) is preferably about 50 mL to about 1000 mL, more preferably about 100 mL to about 900 mL, about 100 mL to about 500 mL, about 150 mL to about 400 mL, about 200 mL to about 300 mL, or about 250 mL.

Preferably, the microcarrier density is about 2 to about 8 $cm^2$/mL, about 2.5 to about 7.5 $cm^2$/mL about 3 to about 7.2 $cm^2$/mL, about 3.6 to about 6 $cm^2$/mL, about 4 to about 5.4 $cm^2$/mL, about 4 to about 5 $cm^2$/mL, about 4.5 to about 5 $cm^2$/mL, or about 4.8 $cm^2$/mL, or about 2.5 to about 4.9 $cm^2$/mL, or about 3.6 to about 4.9 $cm^2$/mL. In a preferred embodiment, the microcarrier density is below 8 $cm^2$/mL, more preferably below 7.2 $cm^2$/mL, more preferably below 5 $cm^2$/mL

Stable growth (under the tested conditions (as e.g. described in the Examples)) was in particular observed between 2.5 and 7.5 $cm^2$/ml, whereas higher concentrations resulted in impaired growth or no growth at al. This observation can be attributed to increased shear stress that occurs due to frequent collisions between microcarriers. Thus, microcarrier density is an important parameter for successful growth of SMDC.

In a particularly preferred embodiment of the present invention, the surface area provided by the microcarriers is about 900 to about 1500 $cm^2$ in a volume of about 250 mL. For higher or lower volumes, the respective relation is preferably adapted accordingly.

In a preferred embodiment of the present invention, the growth surface area for the cells, the volume of the suspension and the microcarrier density of steps (b) are the same as outlined for step (a).

The microcarriers used in the method of the present invention are designed in that CD56 expressing SMDCs are able to attach to them, to grow on them and to be able to be detached from them e.g. by enzymatic treatment.

Preferably, the microcarriers have a size, preferably a diameter, of about 100 to about 300 $\mu$m, more preferably of about 120 to about 250 $\mu$m, or about 150 to about 210 $\mu$m or about 160 to about 200 $\mu$m.

The density of the microcarrier is preferably about 1000 to about 1200 $cm^3$/g, more preferably about 1010 to about 1180 $cm^3$/g, or about 1020 to about 1170 $cm^3$/g, about 1050 to about 1160 $cm^3$/g, about 1060 to about 1170 $cm^3$/g, about 1020 to about 1120 $cm^3$/g, about 1110 to about 1130 $cm^3$/g or about 1080 to about 1150 $cm^3$/g.

Preferably, the surface area provided by the microcarriers is about 200 to about 5000 $cm^2$/g, more preferably about 300 to about 4500 $cm^2$/g, or about 350 to about 1000 $cm^2$/g, about 360 to about 700 $cm^2$/g, about 450 to about 600 $cm^2$/g, or about 500 to about 550 $cm^2$/g.

Preferably, the microcarrier is microporous. Preferably, the microporosity of the microcarriers is only accessible to small molecules such as phenol red. The microporosity does not allow cells to migrate within the bead. Preferably, the pore diameters of the microcarriers is less than 50 nm or in the range of about 1 nm to about 50 nm, or about 2 nm to about 20 nm. In a further preferred embodiment, the pore diameter of the microcarriers is less than 2 nm, or in the range of about 0.001 nm to about 2 nm, about 0.01 nm to about 2 nm, or about 0.1 to 2 nm.

Preferably, the surface of the microcarrier is smooth. Preferably, said smooth surface is characterized in that the surface does not comprise visible unevenness or imperfections. Preferably, said smooth surface has no visible unevenness or imperfections at a magnification of 2x, 10x, 100x, 500x, 600x, 700x or 800x. Such magnification can be analyzed by a suitable magnification device such as a microscope or an Electron microscope.

The surface of the microcarriers may be charged (anionic or cationic) or uncharged. Preferably, the microcarriers in step (a) comprise a cationic charged surface. The cationic charge may for example be obtained by modifying the surface of the microcarrier, e.g. by amine-modification, e.g. by coating the surface of the microcarrier with a suitable amine-compound such as trimethylamine. Alternatively, they may be obtained by substituting the surface moieties by other suitable positively charged compounds such as positively charged N,N-diethylaminoethyl. Alternatively, the microcarriers in step (a) have no surface charge. They may be coated with vitronectin, such as a copolymer coating of poly(hydroxyethylmethacrylate-co-methacrylic acid-polyethylene glycol tetra oligomer vitronectin polypeptide, e.g. by comprising a *Synthemax II* coating (Cornering). Alternatively, said microcarriers may be coated with a mammalian protein such as collagen, gelatin or laminin. In a preferred embodiment, the microcarriers are not coated with a mammalian protein such as collagen, gelatin or laminin.

Preferably, the microcarriers are composed of inert material which is sterile or sterilizable. In a preferred embodiment, the microcarriers are non-dissolvable, i.e. they are preferably not degradable by suitable means for dissolving microcarriers in the art such as enzymes as e.g. pectinase, or change in culture conditions such as temperature or pH value.

Preferably, the microcarrier is composed of a polymaterial or a polymer such as polystyrene, PGA (Poly glycolic acid), PVA (Polyvinyl alcohol), dextran, cotton cellulose, PVA, which may be cross-linked or non-cross-linked. In a particular preferred embodiment, the microcarrier is composed of normal polystyrene (non-cross-linked), cross-linked dextran or PGA, most preferred of non-cross-linked polystyrene.

Particularly preferred are microcarriers comprising a hydrophobic core such as a polymer core, preferably a styrene core. The surface of the microcarriers comprises preferably a cationic moiety. Said cationic moiety is preferably a positively charged hydrophilic moiety. The hydrophilic moiety comprises preferably heteroatoms such as oxygen, sulfur, nitrogen, and phosphorus. Examples include functional groups like alcohol, thiol, ether, ester, acid, sulfate, sulfonate, phosphate, amine, and amide. The cationic moiety may for example be applied to the hydrophobic core of the microcarrier by coating or printing. In a preferred embodiment of the present invention, the microcarriers comprise a polymer core, preferably a polystyrene core, coated with a cationic amine moiety. Examples of suitable cationic amine moieties are moieties comprising trimethylamine such as the Hillex II microcarriers (Sartorius), the PureCoat Amine Surfaces (Cornering) and Poly-D-Lysine coatings.

Particularly preferred are microcarriers comprising a polystyrene core coated with trimethylamine such as the Hillex II microcarriers (Sartorius). Other preferred microcarriers are microcarriers comprising polystyrene with a coating comprising vitronectin, such as a copolymer coating of poly(hydroxyethylmethacrylate-co-methacrylic acid-polyethylene glycol tetra oligomer vitronectin polypeptide, or Synthemax II microcarriers (Corning), or microcarriers comprising cross-lined dextran with a cationic charged surface, such as a surface of a hydrophilic DEAE exchanger such as Cytodex 1 (Cytiva).

The culture medium is preferably a cell culture medium suitable for animal, mammalian or human cell culture. In particular, it is adapted to promote cell adhesion to the microcarriers, cell growth and cell expansion, preferably without allowing the cells to fuse into myofibers or to convert in a different lineage such as smooth muscles. In a preferred embodiment, the culture medium is an FCS-based growth medium which may optionally comprise antibiotics such a gentamycin and growth factors such as basic fibroblast growth factor (bFGF, or FGF-2). In a preferred embodiment, the culture medium does not comprise dexamethasone.

The container is preferably a bioreactor allowing an automated or semi-automated process which enables GMP compliant manufacturing of CD56 expressing SMDCs. In a preferred embodiment, the bioreactor is designed to provide a constant volume. In a further preferred embodiment, the wall of the bioreactor is made of a form-stable material, which is preferably inflexible and/or stiff. Accordingly, the bioreactor is preferably not a bag composed of flexible material. Examples of suitable bioreactors for performing the method according to the present invention are the *Ambr® 250 high throughput multi-parallel bioreactor system* by Sartorius, and the *DASBox stackable mini-bioreactor system* by Eppendorf.

Step (a) of the method according to the invention may comprise one or more preliminary incubation steps. For example, the microcarriers in step (a) may first be incubated in growth medium before the growth medium is inoculated with cells. Such an incubation is preferably performed at the same or similar conditions as used in step (b). By this procedure, the microcarriers are adapted to the culture conditions of step (b) which could enhance the attachment of the cells to the microcarriers. Alternatively, the microcarriers may be added to a suspension of cells and culture medium or the microcarriers and cells may be added to the culture medium at the same time.

In a preferred embodiment, after step (a) and before step (b) a step (b') is performed of cultivating the suspension in the container under conditions allowing the provided cells, in particular CD56 expressing SMDCs, to attach to the microcarriers. Such cultivation is preferably performed for 10 minutes to about 8 hours, more preferably for about 30 min to about 3 hours, for about 40 min to about 2 hours, or for about 50 min to about 100 min, most preferably for about 60 min to about 90 min. It is preferably performed at a temperature in the range of about 25°C to about 40°C, more preferably of about 30°C to about 39°C, of about 35°C to about 38°C, most preferably of about 36 to about 38°C or of about 37°C and preferably under supply of $CO_2$, preferably of about 2 to about 15% $CO_2$, of about 4 to about 10% $CO_2$, of about 4 to 6% $CO_2$, most preferably of about 5% $CO_2$. Preferably, said cultivation is performed statically, i.e. without any mixing, movement, stirring or rocking or by slow mixing, movement, stirring or rocking as e.g. in the range of 5 to 40 rpm, more preferably of 10 to 30 rpm or at about 20 rpm.

Step (b) is preferably performed under conditions allowing the cells adhered to the microcarriers to expand. Preferably, step (b) is also performed under conditions allowing cells, in particular CD56[+] SMDCs, to adhere to the microcarriers. Said conditions are preferably more favorable for CD56$^+$ SMDCs than for CD56$^-$ SMDCs and the remaining cells, respectively. Thus, the cultivation in step (b) is performed under conditions, wherein the ratio of CD56 expressing SMDCs to CD56 non-expressing SMDCs is constant or increased. This means that the ratio of CD56 expressing SMDCs to CD56 non-expressing SMDCs at the end of step (b) is the same or higher as the ratio of CD56 expressing SMDCs to CD56 non-expressing SMDCs at the beginning of step (b) or of the SMDCs provided in step (a). Any deviation less or equal to 10%, 9%, 8%, 7%, 6%, 5%, 4%, 3%, 2%, 1%, or 0% is preferably considered as being constant or the same. Thus, the proportion of CD56$^+$ cells in the expanded SMDCs obtained in step (b) is at least as high (+/- 10%, 9%, 8%, 7%, 6%, 5%, 4%, 3%, 2%, 1%, or 0%), but preferably higher, than the proportion of CD56$^+$ cells in the SMDCs provided in step (a). In a preferred embodiment, the proportion of CD56 expressing cells in the total cell number in step (b) decreases at most by 10%, 9%, 8%, 7%, 6%, 5%, 4%, 3%, 2%, 1% or 0% during expansion. Preferably, at least 60%, more preferably at least 70%, 80% or 90% of the expanded population of SMDCs obtained in step (b) express CD56.

In a further preferred embodiment, the cultivation in step (b) further results in that the proportion of CD49f expressing cells in the total cell number stays the same or preferably even increases during expansion. This means that the proportion of CD49f expressing SMDCs in the total cell number at the end of step (b) is the same or preferably even higher than the proportion of CD49f expressing SMDCs in the total cell number at the beginning of step (b) or of the SMDCs provided in step (a). In a particular preferred embodiment, the cultivation in step (b) results in that the proportion of CD49f expressing cells in the total cell number of SMDCs increased during expansion, preferably by more than 2%, 3%, 4%, or 5 %. Preferably, at least 80%, more preferably at least 90% or 95% of the expanded population of SMDCs obtained in step (b) express CD49f. Presumably, said increased expression of CD49f is caused by the CD56$^+$ cell's adaptation to the 3D culture conditions of the method according to the present invention.

The mixing in step (b) is preferably performed by stirring the suspension. Alternatively, the mixing may be achieved by shaking, tilting or rocking the container. It should be high enough to keep possibly all microcarriers floating but not too high which might result in damaging the cells by shear stress. The mixing of the suspension seems not only to be important for providing similar cultivation conditions to all cells such as nutrients or to avoid or reduce aggregation of cells and/or microcarriers. It was further observed that the stirring speed determines the attachment of the CD56 expressing SMDCs to the microcarriers. Moreover, the inventors of the present invention have surprisingly found that the culture conditions of the method according to the present invention, presumably amongst others due to the mechanical stimulation of the cells provided by the mixing, enhances cell growth, expansion and further properties such as adhesion of the CD56 expressing SMDCs. For example, it seems that mechanical stimulation under the conditions and set up of the method according to the present invention enhances the enrichment of CD56 expressing SMDCs over skeletal muscle derived cell not expressing the cell marker CD56 and/or increases AChE-activity of the expanded CD56 expressing skeletal muscle cells. It seems also to influence the expression of other cell markers such as CD49f as CD49f expression of the SMDCs obtained from 3D culture is increased whereas CD49f expression of SMDCs obtained from 2D culture is decreased.

The mixing may be performed continuously in step (b) with optionally short static phases or phases with increased or decreased mixing. The stirring of the suspension in step (b) is preferably performed at 30 rpm to about 160 rpm, more preferably at about 45 to about 150 rpm, more preferably at about 60 to about 120 rpm, more preferably at about 80 to about 100 rpm, or at about 90 rpm. The stirring speed as outlined above has been determined for a suspension volume of about 250 mL. If the volume in step (b) is significantly more or less than 250 mL, the stirring speed should be adapted accordingly. This can be easily done by a person skilled in the art based on the teaching provided herein and in the Examples. The selection of an appropriate stirring speed is an important parameter in the cultivation of cells and microcarriers. The optimal stirring speed should be determined in a manner that minimizes shear forces while simultaneously preventing excessive clumping of cells and microcarriers, which can lead to localized nutrient deprivation. For example, for the cultivation of SMDCs using 125 ml spinner flasks, a stirring speed of 45 rpm was determined to be suitable. It is important to note that the formation of cell-microcarrier agglomerates was observed during the cultivation process. Despite this observation, no significant negative impact on viability or quality parameters was detected. However, to avoid or reduce cell-microcarrier agglomerates, the stirring speed may be increased several times for a suitable time range. Preferably, the stirring speed is increased by a factor of about 1.2 to about 2, more preferably of about 1.3 to about 1.9, or of about 1.4 to about 1.8, or about 1.5 to about 1.7, or about 1.5 to about 1.6. The stirring speed is preferably reduced to the former stirring speed after observing deagglomeration or after a time range of 30 seconds to about 5 minutes, or of about 40 seconds to about 4 minutes or about 50 seconds to about 3 minutes or about 45 seconds to about 2 minutes, or about 30 seconds to about 2 minutes or about 30 seconds to about 90 seconds or about 45 seconds to about 75 seconds, or for about 60 seconds. In particular preferred embodiment, the stirring speed is increased for about 30 seconds to about 90 seconds from about 80 rpm to about 110 rpm to about 130 to about 150 rpm, or for about 1 minute from about 90 rpm to about 140 rpm. The increase of the stirring speed is preferably performed 1 to 5 times a day, or 1 to 3 times a day. The increase of the stirring speed may be started on the first, second, third, fourth or fifth day of culture. Preferably, it is started on the second day of culture. The stirring parameters as described herein are preferably adjusted to the specific culture vessel, as the form and geometry of the impeller and vessel can impact the flow conditions. Preferably, the stirring is performed counterclockwise. Short intervals of decreased mixing may be performed at about 10 to about 30 rpm, more preferably at about 15 to 25 rpm. Said short intervals are preferably no longer than 2 hours, more preferably no longer than 1 hour. Preferably, said short intervals are performed at most every 12 hours, more preferably at most every 24 hours.

The ratio of rpm to volume of suspension is preferably 0.2 to about 0.5 rpm/ml, more preferably about 0.3 to about 0.45 rpm/ml, more preferably about 0.32 to about 0.4 rpm/ml.

The cultivation in step (b) is performed in a given volume. This means that the volume of the suspension in step (b) remains basically the same and stays within 10%, 5%, 3%, 2% or 1% of the given volume. Any temporary decrease for medium changes does not deviate from this condition. Thus, the cultivation in step (b) in a given volume may comprise temporary volume decreases for changing the culture medium. Details for performing such a medium change are further outlined below. Preferably, the volume at the start and end of step (b) is the same, i.e. the starting volume in step (b) corresponds to the given volume. In a preferred embodiment of the present invention the volume in step (b) is not increased above the starting volume, in particular not above 10%, 5%, 3%, 2% or 1% of the starting volume.

A bead to bead transfer of expanded CD56 expressing SMDCs has successfully been tested, i.e. expanded cells are able to attach to newly added microcarriers during step (b). Thus, during step (b) further microcarriers might be added to the suspension of step (b) for increasing the surface area for the expanded cells. However, the inventors have surprisingly found that the addition of further microcarriers is not necessary for obtaining a sufficient amount of CD56 expressing SMDCs and that it is possible to start initially with the full amount of surface area needed. In fact, the process seems to be safer if no additional microcarriers are added during the expansion phase as e.g. the risk of contamination is reduced. Thus, in a preferred embodiment of the present invention, the microcarrier density in step (a) and/or step (b) remains basically the same. Thus, in a preferred embodiment of the present invention, no additional microcarrier are added in steps (a) or (b) or in between steps (a), (b), or (c).

In step (b) the culture medium is changed several times. Preferably, it is changed at least 3 times, more preferably at least 4, 5, 6, 7, 8, 9, 10, 11, 12, 13, 14, 15, 16, 17, 18, 19 or 20 times. Preferably, the medium is changed regularly. Preferably, not the complete medium is changed but only a part thereof. Preferably, each time about 5% to about 50% of the medium are replaced by fresh culture medium, or about 8% to about 30%, or about 10% to about 20% of the medium. Preferably, the longer the cultivation time, the more often the medium is changed. Preferably, on day 1 and 2 of the cultivation the media change is performed 0-2 times, on day 3 and 4 of the cultivation the media change is performed 1-3 times and from day 5 on the media change is performed 2-4 times. More preferably, on day 1 and 2 of the cultivation the media change is performed once, on day 3 and 4 of the cultivation the media change is performed twice and from day 5 on the media change is performed thrice a day. In a particularly preferred embodiment, about 10 to about 20% of the medium are replaced by fresh culture medium every 24 hours from day 1 to 2, about 10 to about 20% of the medium are replaced by fresh culture medium every 12 hours from day 3 to 4, and about 10 to about 20% of the medium are replaced by fresh culture medium every 8 hours from day 5 on. The medium change is preferably performed by first removing parts of the medium from the container and subsequently adding fresh medium until the given volume is reached again. Each medium change typically takes about 1 to about 30 minutes, more preferably about 4 to about 20 minutes or about 10 to about 15 min. Preferably, the mixing is continued during the media change.

The total medium consumption during cultivation is preferably 2-fold to 5-fold of the medium volume within the bioreactor, more preferably about 2.5-fold to about 4-fold, or about 2.8 fold to about 3.9 fold.

Preferably, step (b) is performed until a cell number of about 40 million cells to about 200 million cells is obtained or of about 40 million cells to about 180 million cells, about 40 million cells to about 60 million cells, about 40 million cells to about 120 million cells, about 80 million cells to about 120 million cells, about 80 million cells to about 200 million cells or about 80 million cells to about 180 million cells.

Preferably, cells are expanded by the method according to the present invention by a factor of about 20 to about 30, or of about 22 to about 28, about 23 to 27 or about 24 to 26. Thus, the cell number of cells obtained in step (b) is preferably 20 to about 30 times as high as the cell number of cells provided in step (a).

Preferably, step (b) is performed until a cell density of about $1.6 \times 10^5$ cells/ml to about $8 \times 10^5$ cells/ml is obtained or of about $1.6 \times 10^5$ cells/ml to about $7.2 \times 10^5$ cells/ml, about $1.6 \times 10^5$ cells/ml to about $2.4 \times 10^5$ cells/ml, about $1.6 \times 10^5$ cells/ml to about $4.8 \times 10^5$ cells/ml, about $3.2 \times 10^5$ cells/ml to about $4.8 \times 10^5$ cells/ml, about $3.2 \times 10^5$ cells/ml to about $8 \times 10^5$ cells/ml or about $3.2 \times 10^5$ cells/ml to about $7.2 \times 10^5$ cells/ml. The production of higher cell numbers is associated with a higher risk of cell alterations and is therefore preferably avoided. Such cell alterations represent a potential risk if the obtained cells are intended for medical uses such as implantations into subjects in need thereof. Step (b) is preferably performed for about 4 to about 12 days, for about 5 to about 12 days, more preferably for about 4 to about 10 days, for about 6 to about 10 days, for about 4 to about 8 days, for about 4 to about 7 days, for about 5 to about 7 days, or for about 5 to about 9 days.

In a preferred embodiment of the present invention, step (b) comprises the monitoring of premature fusion effects. This is preferably done by live cell imaging, immunostaining on skeletal muscle specific myosin heavy chain (fast-twitch fiber), measuring AChE activity or other suitable means. Measuring of AChE activity may either be performed by measuring the AChE activity of the cells in culture or by measuring AChE activity in the supernatant. An experiment with differentiated cells demonstrated that supernatants from both 2D and 3D cultures produced higher AChE activity values than undifferentiated cells.

In optional step (c), the expanded cell population attached to the microcarriers is harvested. Step (c) is preferably performed by detaching the cells from the microcarriers. In a preferred embodiment, step (c) is performed enzymatically. Alternatively, the cells are detached by other common means known in the art. One example, is the use of microcarriers which are dissolvable. Thus, step (d) may be performed by dissolving the microcarriers, e.g. by a change in temperature or medium conditions or by dissolving the microcarriers enzymatically.

The harvest of the cells is performed after the desired cell number and/or cell density has been reached. In a preferred embodiment, the method of the present invention comprises a step of detaching the cells from the microcarriers at the end of the cultivation by cleaving the anchorage proteins via enzymatic or mechanical means. Moreover, step (c) may comprise a step of retrieving the cells from the bioreactor.

In a preferred embodiment, step (c) is performed enzymatically by use of one or more suitable enzymes, such as a protease, such as an endo- or exoprotease, an endopeptidase or other proteolytic enzymes that catalyses the hydrolytic cleavage of peptide bonds necessary for cell anchorage. Preferred enzymes are trypsin, papain, collagenase, pronase, dispase or pectinase or combinations thereof, as e.g. a combination of trypsin and pronase or trypsin and pectinase or trypsin and organic acid EDTA (ethylenediaminetetraacetic acid). Preferably, said enzymes are recombinant enzyme and thus animal-component-free enzymes. Particularly preferred are recombinant proteases or recombinant endopeptidase which are able to break down proteins that enable cell adhesion to the surface e.g. by cleaving at the C-terminal side of lysine and arginine, thereby disrupting the bonds that hold cells in place. Particularly preferred are recombinant Trypsin alternatives or animal-component-free trypsin substitutes such as *TrypLE select* (Thermo Fisher Scientific Inc., MA, USA).

Said one or more enzymes may be added to the bioreactor after step (b). It/they are preferably added after the culture medium has been removed. Preferably, the culture medium is replaced by a solution comprising the one or more enzymes. Alternatively, the microcarriers may be first retrieved from the bioreactor and resuspended in a solution comprising the one or more enzymes. After removing the culture medium and prior to the addition of the one or more enzymes, step (c) may comprise one or more washing steps, most preferably 1 to 3 or 2 washing steps. Said washing may be performed with a suitable buffer such as PBS.

After the cells have been detached from the microcarriers, they are preferably separated from the microcarriers by filtration, preferably vacuum filtration. In a particular preferred embodiment, the cells are separated from the microcarriers by using one or more suitable strainers such as cell strainers, more preferably by two or more strainers having distinct pore sizes. The first strainer has preferably a pore size of about 30 to 120 $\mu$m, more preferably of about 100 $\mu$m, about 70 $\mu$m or 40 $\mu$m. The second strainer has preferably a pore size of about 5 to 25 $\mu$m, more preferably of about 10 to about 20 $\mu$m, most preferably of about 15 $\mu$m. The second strainer is preferably stocked beneath the first strainer. In case of dissolvable microcarriers, the cells are preferably harvested by centrifugation.

In a preferred embodiment, the expanded population of SMDCs obtained in step (b) and/or (c) comprise more than 90% viable cells, more preferably more than 93%, 94 %, 95%, 96%, 97%, 98% or 99% viable cells.

Preferably, the cells in step (a) are a mixture of different kind of cells obtained from skeletal muscle tissue. For example, they may differ in their expression of cell markers. In particular, said cells may comprise CD56 expressing cells and cells not expressing the cell marker CD56. Skeletal muscle derived cells expressing CD56 are preferably myogenic and/or fusion competent. Skeletal muscle derived cells not expressing CD56 are preferably non-myogenic and/or non-fusion competent. Preferably, the cells in step (a) comprise at least 30% CD56 expressing cells, more preferably at least or about 40%, 50%, 60%, 70%, 80%, 90%, 95% or 98% CD56 expressing cells. In a preferred embodiment, the ratio of the amount of CD56 expressing cells to CD56 non-expressing cells is about 30 to about 70, about 40 to about 60, about 50 to about 50, or about 60 to about 40, or about 70 to about 30, or about 80 to about 20, or about 90 to about 10.

Preferably, if tested, the SMDCs provided in step (a) have an AChE activity of at least 50 mUrel per $2\times10^5$ cells following five to seven days of differentiation. Preferably, if tested, the expanded population of SMDCs obtained in step (b) have also an AChE activity of at least 50 mUrel per 200 000 cells following five to seven days of differentiation.

Preferably, the SMDCs provided in step (a) are cells which have been obtained from the skeletal muscle tissue sample by isolating cells from said muscle tissue sample. The cells in step (a) are preferably mammalian cells, preferably from a human, a mouse, or a pig, most preferably from a human.

Even though it has been shown that CD56[+] cells can be enriched using the method according to the invention, the expansion according to the invention is particularly successful if a large number of CD56 cells are already used in step a). In this respect, the population of SMDCs is preferably provided by a method that already provides many CD56[+] cells as starting material. This may, for example, be necessary, if the sample obtained from skeletal muscle tissue is of minor quality or comprises only few CD56[+] cells (e.g. due to an advanced age of the subject from which the skeletal muscle tissue was obtained). Thus, in a preferred embodiment, the SMDCs in step (a) may be obtained by a method providing a high amount of CD56[+] expressing cells as outlined in the following.

The SMDCs in step (a) may preferably be obtained by a method comprising the following steps:

(i) cooling of a sample obtained from skeletal muscle tissue in a liquid;

(ii) processing of the sample and cooling the processed sample; and

(iii) resuspending the processed sample of step (ii) in a mixture comprising one or more enzymes under conditions allowing an enzymatic digestion of the processed sample by said one or more enzyme(s), thereby obtaining a suspension comprising single cells and remaining tissue fragments.

The liquid in step (i) is preferably a buffer, a solution, a transport solution, a nutrient mixture, a saline solution, a medium, or cell culture medium such as Ham's F10. Said liquid is suitable for storing, transporting and/or preserving the tissue and to keep the cells of the tissue alive. It may comprise additive for improving preservation of the tissue such as antibiotics to prevent bacterial contamination.

In a preferred embodiment of the present invention, the skeletal muscle tissue in step (a) is obtained from a muscle biopsy. Such muscle biopsy serving as the source of skeletal muscle-derived cells, in particular of CD56 expressing SMDCs, can be obtained from the muscle at the site of injury or from another area that may be more easily accessible to the clinical surgeon. The site of the biopsy is not restricted to a distinct skeletal muscle, and may be such as from the upper arm. The size of the biopsy may comprise approximately 1 cm x 1 cm x 1 cm or bigger.

For using CD56 expressing SMDCs in the treatment of muscle injuries, for example for the treatment of incontinence, said SMDCs are preferably isolated from a skeletal muscle biopsy of the subject to be treated. Thus, said SMDCs are preferably autologous to the subject.

In a further preferred embodiment, step (i) is conducted at a temperature lower than 16°C, preferably at a temperature range from 1 to 16°C, preferably 4 to 10°C, 6 to 8°C, or 1 to 3°C; Further, it is preferred that the method of the present invention in step (i) is conducted at a time in the range of up to 96 hours, or any time within this range, such as 12 to 96 hours, 12 to 72 hours, 12 to 48 hours, 24 to 96 hours, 24 to 72 hours, 24 to 48 hours, or any other intermediate range.

Preferably, step (ii) comprises a mechanical disruption of said tissue sample. Such a mechanical disruption is necessary for obtaining specific cells of said sample. It preferably comprises the separation of muscle tissue from connective tissue and/or the dissection into smaller pieces, mincing and/or shredding of the skeletal muscle tissue. Such mechanical disruption is usually performed at room temperature. The processing of the sample comprises preferably the use of scissors, scalpel, tweezers, filter, or ball mill and a centrifuge.

After the processing of the sample, the processed sample is preferably cooled. The cooling is preferably conducted at a temperature of the sample in the range of 1 to 16°C for a time in the range of 2 to 48 hours. More preferably, it is conducted at a temperature in the range of 1 to 16°C or at any temperature in between this range, such as at 2, 3, 4, 5, 6, 7, 8, 9, 10, 11, 12, 13, 14, 15°C, or any intermediate temperature within this range. In a further preferred embodiment of the present invention, step (ii) is conducted at a temperature in the range from 2 to 8°C or 4 to 8°C, particularly preferred at 4°C. In a further preferred embodiment, the temperature range is below 4°C, preferably in the range from 1 to 3°C.

Further preferred, step (ii) of the present invention is conducted for a distinct time range, such as 2 to 60 hours, or any time within this range, such as 2 to 60 hours, 8 to 60h, 16 to 60h, 24 to 60h, 36 to 60h, 40 to 52h or any other intermediate range. The advantages of cooling the processed sample are in detail described in WO 2019/115790.

Preferably, it is foreseen that step (iii) comprises conducting the enzymatically treatment with a solution comprising any one or more selected from the group consisting of trypsin, papain, elastase, hyaluronidase, collagenase, and dispase. In a further preferred embodiment, step (iii) is performed in medium such as a cell culture medium, preferably comprising a serum. Preferably, step (iii) is performed with a supply of 5% $CO_2$.

In a preferred embodiment, the mixture comprises preferably at least one enzyme, most preferably collagenase. In a further preferred embodiment, the mixture comprises preferably at least a second enzyme, such as Trypsin or a recombinant alternative such as *TrypLE select.* The enzymatic treating in step (iii) is preferably performed by heating the mixture up to about 25 to about 38°C, preferably up to 36 to 38°C, particularly preferred up to 37°C. The enzymatic treatment is preferably conducted for about 10 min to 20 hours, about 10 min to about 1 hour, about 10 min to about 30 min, about 1 hour to about 20 hours, about 2 hours to about 20 hours, about 5 hours to about 20 hours, about 6 hours to about 20 hours, about 8 hours to about 20 hours, about 12 hours to about 20 hours or about 16 hours to about 20 hours.

In accordance with the present invention, it is preferably foreseen that resuspending in step (iii) comprises the centrifugation of the sample of step (ii), discarding the supernatant and resuspending of the cell pellet in the mixture comprising at least one enzyme.

After the enzymatic treatment in step (iii), the enzyme(s) used in step (iii) are preferably inactivated by dilution, addition of serum and/or pelleting the obtained suspension and subsequent resuspension.

The suspension comprising single cells and remaining tissue fragments obtained in step (iii) may optionally be passed through one or more cell strainers for separating the tissue fragments from the single cells. Preferably one, two, three or four cell strainers are used having a suitable mesh size for separating the tissue fragments from the single cells. Suitable mesh sizes include for example mesh sizes of about 90 to 110 μm, 30 to 60 μm and 10 to 20 μm. Preferred is a series of three cell strainers having mesh sizes of 100, 40 and 15 μm. This procedure is particularly preferred if the single cells obtained in step (iii) are provided as SMDCs in step (a) of the present invention. However, if after step (iii) optional step (I), as described below, is performed, the suspension obtained in step (iii), which comprises single cells and remaining tissue fragments, is preferably directly plated in step (I) of the present invention.

In a further preferred embodiment, step (iii) is performed by resuspending the processed sample of step (ii) in a mixture comprising at least a first enzyme, heating the mixture up to about 25 to about 38°C, more preferably to about 37°C for about 1 to 20 hours, more preferably for about 6 hours to about 20 hours, in a first incubation step. The mixture for the first incubation step is preferably an enzymatic solution comprising the first enzyme in a suitable buffer or in culture medium optionally comprising serum. In a preferred embodiment, the enzymatic solution comprising the first enzyme comprises culture medium (such as Hams's F10), a buffer (such as HEPES) and serum (such as FCS, preferably at most 10% FCS). The first enzyme is preferably a protease, more preferably an endopeptidase, most preferably collagenase. After said first incubation step, the at least first enzyme is preferably inactivated, for example by pelleting the sample, by diluting the sample and/or by adding a compound, composition or medium inactivating the at least one enzyme. After said first incubation step or after the inactivation of the at least first enzyme, the processed sample is preferably incubated in a second incubation step in a mixture comprising at least a second enzyme. Said second enzyme is preferably a distinct protease, more preferably an endopeptidase, most preferably Trypsin or a recombinant protease or endopeptidase such as a recombinant alternative to Trypsin such as *TrypLE select.* Preferably, said second incubation step is performed by a second enzyme which is applied in a higher activity. Said higher activity results in further decreasing the size of the remaining tissue fragments. The second incubation step is preferably performed by heating the mixture up to about 25 to about 38°C, or to about 36 to about 38°C, or to about 37°C for about 5 to about 30 minutes, more preferably to about 5 to about 15 minutes, more preferably for about 10 minutes. The mixture for the second incubation step is preferably a suitable enzyme solution for digesting the sample with the second enzyme and may comprise an EDTA and/or NaCl solution or PBS solution. For example, concentrated Trypsin (e.g.10x concentrated) may be solved in a NaCl solution comprising EDTA. In a preferred embodiment the mixture for the second incubation step comprises no cell culture medium and/or no serum. After said second incubation step, the at least second enzyme is preferably inactivated, for example by pelleting the sample, by diluting the sample and/or by adding a compound, composition or medium inactivating the at least one enzyme. For example, the enzyme is inactivated by the addition of cell culture medium comprising serum.

The inventors of the present invention have surprisingly found that the performance of a second incubation step with a second enzyme results in a higher cell yield which is beneficial for primary culture initiation. In general, the enzymatic digestion results in that the processed sample is digested in single cells and remaining tissue fragments. After the 2nd digestion step, these fragments are much smaller than without, so the ratio between single cells and fragments is shifted more towards single cells. The higher cell yield results in that the CD56 expressing SMDCs have to divide less often for obtaining the required cell number. This may help that the ratio of CD56 expressing SMDCs to CD56 non-expressing SMDCs in step (b) of the method according to the present invention and the proportion of CD56 expressing SMDCs in the total cell number obtained in step (b), respectively, is constant or increased.

In a preferred embodiment of the present invention, the SMDCs provided in step (a) have prior to step (a) be cultured in a 2D culture as a so called primary culture. Thus, before expansion in 3D culture on microcarriers is started in step (b), the method according to the present invention comprises preferably the following steps:

(I) plating skeletal muscle derived cells (SMDCs) obtained from skeletal muscle tissue onto a dish and incubating the dish under conditions allowing cells including CD56 expressing SMDCs to adhere to the surface of the dish,
(II) propagating the adherent cells including CD56 expressing SMDCs obtained in step (I), and
(III) detaching the cells including CD56 expressing SMDCs obtained in step (II) from the surface of the dish.

In a preferred embodiment the present invention, methods steps (I), (II) and (III) are performed after step (iii), if the SMDCs provided in step (a) have been obtained by a method comprising steps (i), (ii) and (iii). In that case, the suspension of single cells obtained in step (iii) is plated onto a dish in step (I) and the detached cells obtained in step (III) are provided as SMDCs in step (a) of the method according to the present invention.

Preferably, incubation in step (I) is conducted at a temperature in the range of 25 to 38°C, preferably 36 to 38°C, in particular preferred at 37°C, thereby obtaining adherent cells including CD56 expressing SMDCs. This further incubation step allows to let the cells grow to achieve a higher amount of SMDCs. A person skilled in the art will adjust the culture conditions, such as medium and temperature, accordingly to gain a maximum amount of SMDCs. Step (I) may for example be performed in standard growth medium, optionally added by antibiotics such as gentamycin. During the incubation, the medium may be changed. Preferably, said medium change results in that non-myogenic cells or cells which are floating in the culture container, such as dead cells, or other residuals such as remaining tissue fragments of the suspension obtained in step (iii) are discarded. The first medium change may be performed after four to 24 hours, the following medium changes may be performed every two to three days. The medium used for the 2D culture may be the same as used for the 3D culture described above. In a preferred embodiment, the medium comprises Hams F10, 10% FCS, growth factors such as bFGF and optionally antibiotics as Gentamicin.

The dish of step (I) is preferably one or more dishes as e.g. 1, 2, 3, 4, 5, 6, 7 or 8 dishes. The dish is preferably a flask, in particular cell culture flask such as a T-75 culture flask or a culture flask having a *CellBIND surface*. The surface of the dish is preferably uncoated or coated with laminin or any other factor promoting adherence such as collagen, fibronectin, vitronectin, calcium ions, proteoglycans and ECM polysaccharides or ECM mimetics, in an amount effective to promote adherence. In a particularly preferred embodiment, the surface of the dish is coated with Laminin, a recombinant Laminin, Laminin 521 (such as *Biolaminin LN521 (a* recombinant laminin 521 substrate by Biolamina), *Biolaminin 521* MX (Biolamina), or *Biolaminin 521 CTG* (Biolamina)) or Laminin 511. A recombinant Laminin has the advantage of not having animal origin. The coating with laminin can e.g. be performed by coating them with a laminin solution in a suitable buffer (e.g. DPBS with $^{Ca2+/Mg2+}$) for a sufficient amount of time and at a suitable temperature such as for two hours at about 36 to about 38°C or for several hours as e.g. overnight at about 2 to about 8 °C.

The inventors of the present invention have surprisingly found that Laminin coatings were beneficial for long-term stabilization of the myogenic CD56$^+$ target cell population within the isolated SMDCs. The coating with laminin seems to keep CD56 expression of SMDCs more stable. Moreover, it was found that the myogenic CD56$^+$ target cell population grown on Laminin showed rapid growth without premature fusion events. Furthermore, Laminin coating has demonstrated the ability to enhance the attachment and thereby accelerate the growth of SMDCs in 2D culture while preserving a high myogenic potential. Thus, Laminin coating can reduce the duration of the primary culture (2-D culture) and/or improves the overall quality of the cells obtained.

The propagating in step (II) preferably comprises the culturing of the adherent cells for 1 to 5 passages, more preferably for 1 to 3 passages, or for 1 or 2, most preferably 1 passage, to 70 to 80% confluency. In accordance with the present invention, a person skilled in the art is able to determine the necessary duration and amount of passages to achieve the desired confluency of 70 to 80%. Alternatively, the cultivation in step (II) is performed up to a cell number of about 1 to about 6 million SMDCs, more preferably of about 2 to about 6 million SMDCs.

The detachment in step (III) is preferably done by an enzymatic cell detachment using a suitable enzyme such as those described for step (c) of the present invention. Preferably, Trypsin or a recombinant alternative such as *TrypLE select* is used for the detachment in step (III). *TrypLE select* is an animal-component-free alternative to Trypsin, containing recombinant Protease, which was developed by Thermo Fisher Scientific Inc. The enzymatic detachment is performed under conditions resulting in the detachment of the adhered cells from the surface of the dishes as e.g. for about 1 to about 15 minutes, more preferably for about 5 to about 10 minutes at a temperature of about 25 to about 40°C, more preferably of about 35 to about 38°C. Subsequently, the detached cells are preferably resuspended in medium (preferably culture medium comprising serum).

The inventors of the present invention have surprisingly found that the performance of a primary 2D-culture, as e.g. outlined in steps (I) to (III) above, prior to the 3D-culture improved the performance of the 3D culture.

In a particularly preferred embodiment of the present invention, the cells in step (a) have been obtained by a method comprising the following steps:

(i) cooling of a sample obtained from skeletal muscle tissue in a liquid;

(ii) processing of the sample and cooling the processed sample in the range of 1 to 16°C for a time in the range of 2 to 60 hours, more preferably in the range of about of 2 to 8°C for a time in the range of 24 to about 60 hours or of about 36 to about 60 hours, or about 40 to 52 hours;

(iii) resuspending the processed sample of step (ii) in medium, optionally comprising serum, comprising at least a first enzyme, preferably collagenase, heating the suspension up to about 25 to about 38°C, more preferably to about 37°C for about about 6 to about 20 hours in a first incubation step, pelleting the sample and resuspending the pellet in a mixture comprising at least a second enzyme, preferably Trypsin or a recombinant alternative such as *TrypLE select,* heating the mixture up to about 25 to about 38°C for about 5 to about 15 minutes, thereby obtaining a suspension comprising single cells and remaining tissue fragments;

(I) plating the suspension comprising single cells and tissue fragments obtained in step (iii) onto a dish, preferably coated with a laminin, and incubating the dish under conditions allowing cells including CD56 expressing SMDCs to adhere to the surface of the dish, preferably at a temperature in the range of 25 to 38°C, thereby obtaining adherent cells including CD56 expressing SMDCs;

(II) propagating the adherent SMDCs obtained in step (I), preferably by culturing of the adherent cells for 1 to 2 passages to 70 to 80% confluency or up to a cell number of about 1 to about 6 million cells; and

(III) detaching the cells obtained in step (II) from the surface of the dish, preferably by a suitable enzyme such as Trypsin or a recombinant alternative such as *TrypLE select.*

In a preferred embodiment of the present invention, the cell population detached in step (III) and/or obtained in step (b) comprise at least 30%, 40% or 50%, but more preferably 60%, 70%, 80%, 85%, 90%, 95%, 96%, 97%, 98% or 99% CD56 expressing SMDCs.

In a preferred embodiment of the present invention, the expanded population of SMDCs obtained by the method according to the present invention is fusion competent, multinucleated fusion competent and/or skeletal-myogenic. It has preferably an AChE activity of at least 50 mUrel/ml per 200 000 cells following five to seven days of differentiation, more preferably of at least 60, 80, 100, 120, 140, 160, 180, 200, 220, 240, 260, 280, 300, 320, 340, 360, 380, 400, 420, 440 or 460 mUrel/ml as measured in accordance with Example 7. Preferably, the expanded population of SMDCs obtained by the method according to the present invention has an AChE activity of about 50 to about 450 mUrel/ml, more preferably of about 100 to about 350 mUrel/ml, more preferably of about 200 to about 450 mUrel/ml or of about 50 to about 400 mUrel/ml, of about 100 to about 400 mUrel/ml, or about 200 to about 400 mUrel/ml.

In a preferred embodiment of the present invention, the expanded population of SMDCs obtained by the method according to the present invention comprises at least 60%, more preferably at least 70%, 80%, 85% or 90%, CD105 positive and at least 60%, more preferably at least 70%, 80%, 85% or 90%, CD90 positive cells and is characterized in that said population comprises at least 60%, more preferably at least 70%, 80%, 90% or 95%, CD49f positive cells. In a more preferred embodiment, the expanded population of SMDCs obtained by the method according to the present invention comprises at least 60% CD56 positive, at least 60% CD105 positive and at least 60% CD90 positive cells and at least 80% or 90%, 95% CD49f positive cells or at least 80%, more preferably at least 90%, CD56 positive, at least 80%, more preferably at least 90%, CD105 positive and at least 80%, more preferably at least 85% or 90%, CD90 positive cells and is characterized in that said population comprises at least 80%, more preferably at least 90% or 95% CD49f positive cells.

In a further preferred embodiment, the expanded population comprises at least 50%, more preferably at least 60% or 70%, most preferably at least 80% or 90% alpha-7 (ITGa7) positive cells and/or at least 50% Myogenic factor 5 (Myf5) positive cells. Preferably, at least 50%, more preferably at least 60%, of the cells of the expanded population express the cell marker Desmin. Preferably, at least 50%, more preferably at least 60% or 70%, of the cells of the expanded population express the cell marker SSEA4. Preferably, the cells of the expanded population do not express the cell markers myosin heavy chain (MHC) and smooth muscle actin (SMA) which means that at most 10%, more preferably at most 5% of the cells express cell markers myosin heavy chain (MHC) and smooth muscle actin (SMA). Preferably, the expanded population comprises at least 40 million cells.

In a particularly preferred embodiment, the expanded population of SMDCs obtained by the method according to the present invention comprises at least 80% CD56 positive cells, at least 80% CD105 positive, at least 80% CD90 positive cells and at least 80% CD49f positive cells. Preferably, at least 50% of said cells are Myf5 positive cells. Preferably, at least 80% of said cells are ITGa7 positive cells. Preferably, at least 50% of said cells are Desmin positive and at least 50%, more preferably at least 60%, are SSEA4 positive. Preferably, said cells do not express the cell markers myosin heavy chain (MHC) and smooth muscle actin (SMA).

In a particularly preferred embodiment, the expanded population of SMDCs obtained by the method according to the present invention comprises at least 90% CD56 positive cells, at least 90% CD105 positive, at least 85% CD90 positive cells and at least 90% or 95% CD49f positive cells. Preferably, at least 50% of said cells areMyf5 positive cells. Preferably, at least 90% of said cells are ITGa7 positive cells. Preferably, at least 50% of said cells are Desmin positive and at least 60% or at least 70% are SSEA4 positive. Preferably, said cells do not express the cell markers myosin heavy chain (MHC) and smooth muscle actin (SMA).

Preferably, less than 20%, or less than 15%, 10%, 7%, 5% or 2%, of the population express CD34 and/or MyoD. In a preferred embodiment, the expanded population of SMDCs expresses the cell marker Pax7 but in a more preferred embodiment, less than 20%, or less than 15%, 10%, 7%, 5% or 2%, of the population express Pax7.

Preferably, the methods according to the present do not comprise any steps of cell sorting such as FACS and MACS.

The inventors of the present invention have surprisingly found that comparison between both culture systems in potency assay revealed that 3D culture was superior to 2D culture on average. Moreover, expanded populations of SMDCs obtained from 3D culture according to the present invention showed fusion ability, wherein cells fused to form large multinucleated myotubes.

During 3D culture, the cells are exposed to many stimuli such as mechanical forces due to mixing or the surface of the microcarriers. Said exposure to stimuli results in an adaptation of the cells to said stimuli. For example, the cells adhering to the surface of the microcarriers may comprise adhesion molecules adapted to the specific surface of the microcarriers. This may result in that the adhesion molecules of the expanded CD56$^+$ SMDCs differ from the adhesion molecules of the CD56$^+$ SMDCs provided in step (a). In general, 3D culture enables cells to attach, grow, and interact in a more natural, tissue-like manner than in 2D culture. This approach mimics the in vivo environment more closely. Thus, adaptation to the stimuli of 3D culture may enhance the myogenic properties of said CD56 expressing SMDCs.

In fact, in direct comparison of 3D and 2D cultivation it was surprisingly found that the expanded population of SMDCs obtained by the method according to the present invention showed increased expression of CD49f in comparison to populations of SMDCs expanded in 2D culture. CD49f, also known as Integrin $\alpha$6, is a transmembrane protein widely expressed across various stem cell populations. In myoblasts, CD49f plays a key role in cell adhesion and differentiation, with its absence being linked to impaired myotube formation (Wilschut et al. Stem Cell Res 2011; 7:112-123). The dynamic conditions within the stirred DASbox® bioreactor system may promote increased cell adhesion to the microcarriers, potentially driving the observed upregulation of CD49f. This heightened CD49f expression might also contribute to enhanced fusion potential, as evidenced by the higher acetylcholinesterase (AChE) activity in bioreactor (i.e. 3D)-cultured cells. Moreover, expanded populations of SMDCs obtained by the method according to the present invention showed enhanced myotube formation.

Thus, the method according to the present invention results not only in the expansion of CD56 expressing SMDCs but preferably also in providing an expanded population of CD56 expressing SMDCs having altered or adapted characteristics which improve their abilities to form neuro-muscular connections as well as regenerating muscle weakness e.g. by regenerating skeletal muscle tissue.

A further subject-matter of the present invention is, therefore, directed to an expanded population of SMDCs obtained according to a method of the present invention. Said population has preferably increased or stable CD49f expression in comparison to the SMDCs provided in step (a). Preferably, said population has altered adhesion molecules in comparison to the SMDCs provided in step (a). In a further preferred embodiment, the population of SMDCs expanded in step (b) have enhanced myogenic properties in comparison to the SMDCs provided in step (a).

Thus, the present invention also refers to an expanded population of SMDCs comprising at least 80%, more preferably at least 90% or 95%, CD49f positive cells. Said population comprises preferably at least 40 million cells. In a preferred embodiment, the present invention refers to an expanded population of SMDCs comprising at least 80% CD56 positive, at least 80% CD105 positive and at least 80% CD90 positive cells, characterized in that said population comprises at least 80%, more preferably at least 90% or 95%, CD49f positive cells.

In a preferred embodiment, the expanded population of SMDCs according to the present invention is obtained by the method according to the present invention. Thus, in a preferred embodiment, the expanded population of SMDCs according to the present invention has the same characteristics as the expanded population of SMDCs obtained by the present invention and as outlined in details herein.

A further subject-matter of the present invention is directed to the expanded population according to the present invention or the expanded population of SMDCs obtained according to a method of the present invention for use in a method for prevention and/or treatment of the human or animal body by surgery or therapy or as a pharmaceutical composition. In accordance with the present invention, the expanded population of SMDCs can be used as active ingredient within a pharmaceutical composition.

A further subject-matter of the present invention is directed to the expanded population according to the present invention or the expanded population of SMDCs obtained according to a method of the present invention for use in a method of preventing and/or treating neuromyopathies, myopathies and/or muscle dysfunctions. Preferably, said neuromyopathies and/or myopathies are or result in incontinence such as fecal incontinence and/or urinary incontinence. Preferably, said muscle dysfunction is a skeletal muscle dysfunction such as incontinence, preferably fecal incontinence and/or urinary incontinence.

In accordance with the present invention, the expanded population of SMDCs may be used in a method which preferably foresees that the SMDCs are injected in a subject suffering from incontinence. In general, injecting SMDCs into a given tissue or site of injury comprises a therapeutically effective amount of cells in solution or suspension, preferably of about $1 \times 10^5$ to about $6 \times 10^6$ cells per 100 $\mu$l of injection solution. The injection solution is a physiologically acceptable medium, with or without autologous serum. Physiological acceptable medium can be by way of non-limiting example physiological saline or a phosphate buffered solution.

In principle, any type of anal incontinence can be treated, as the strengthening of the anal muscle systems provides for a better control of the rectal filling. However, anal incontinence that results from perineal rupture, especially if the anal sphincter system and/or if the M. puborectalis is damaged and injured, is treated. Such perineal rupture can result from a broad variety of causes as outlined above. However, the cause of such perineal rupture is not in limiting for the application of the methods and SMDCs of the present invention. Patients may be treated with the methods and SMDCs of the present invention if they suffer from a perineal rupture of the third or fourth grade. This applies in particular to women which suffer from such perineal rupture after forceps delivery, give birth for the first time, deliver a child of a weight over 4 kg, or suffer from a consequence due to position anomalies of the child before birth. The methods and SMDCs of the present invention can also be applied after injury of the anal sphincter system and/or M. puborectalis due to surgical procedures. Additionally, the methods and SMDCs of the present invention can also be applied if there is only transient incontinence. Such treatment prevents development of full anal incontinence. Further anal incontinence disease states to be treated with the methods and SMDCs of the present invention are passive incontinence, faeces incontinence and imperative defecation.

It should be understood that the methods and population of SMDCs according to the present invention cannot only be used to treat patients already suffering from anal incontinence, i.e., showing symptoms of anal incontinence, but may be applied to subjects not yet suffering from anal incontinence, but with increased risk of doing so, for example, in cases where the rectal musculature suffered damage from surgery, birth, accidents and so forth. Another example would be cases where the rectal musculature became thinner than in a healthy individual or where it degenerated due to other reasons. The methods of the present invention can provide a suitable prophylaxis to prevent onset of anal incontinence.

The expanded population of SMDCs for use in the treatment or prophylaxis of incontinence is preferably homologous to the recipient. In a more preferred embodiment said population is autologous or heterologous to the recipient. Autologous SMDCs reduce or minimize the risk of allergic reactions, after the SMDCs have been injected into the recipient.

Therefore, the present invention also provides a simple prophylaxis approach or treatment method for women and men with urinary and/or anal incontinence or in risk of developing urinary and/or anal incontinence by using autologous skeletal muscle-derived cells to enhance their urinary and/or anal sphincters. Such muscle-derived cell therapy allows repair and improvement of damaged urinary and anal sphincter. In accordance with the present invention the treatment comprises a needle aspiration to obtain muscle-derived cells, for example, and a brief follow-up treatment to inject cultured and prepared cells into the patient. Also, according to the present invention, autologous skeletal muscle derived cells (SMDC) harvested from and cultured for a specific urinary and/or anal incontinence patient can be employed as a non-allergenic agent to bulk up the urinary and/or rectum wall, thereby enhancing coaptation and improving the urinary and/or anal sphincter muscle. In this aspect of the invention, simple autologous muscle cell transplantation is performed, as discussed above.

In accordance with the present invention, autologous skeletal muscle-derived cells administered directly into the urinary sphincter and/or anal sphincter, exhibit long-term survival. Thus, autologous SMDCs injection results in safe and non-immunogenic long-term survival of myofibers in the urinary and/or anal sphincter.

In a particular embodiment according to the invention, a population of SMDCs having an effective amount of CD56 expressing SMDCs is injected into the urinary sphincter for treating urinary incontinence or into the anal sphincter, in particular into the external anal sphincter, for treating anal incontinence. Preferably, about 50 to about 200 $\mu$l of suspension comprising the population of SMDCs according to the present invention (with a concentration of about $1 \times 10^5$ to about $6 \times 10^6$ cells per 100 $\mu$l of injection solution) are injected into the urinary sphincter. For the treatment of anal incontinence preferably about 50 $\mu$l to about 1 ml, more preferably about 0.5 ml of a suspension comprising the population of SMDCs according to the present invention (with a concentration of about $1 \times 10^5$ to about $6 \times 10^6$ cells per 100 $\mu$l) are injected into the external anal sphincter.

A particular penetration route is through the skin of a patient in parallel to the course of the rectum. However, it is also contemplated, that the penetration can occur directly from the rectum in the vicinity of the injured muscle. In particular, the penetration and injection process are monitored via sonographic imaging means. Additionally, an alternative penetration route is contemplated for women, that is, trans-vaginal injection. In this scenario, the injection device penetrates the wall of the vagina and is moved forward until it reaches the desired injection site.

In particular, the penetration and injection process are monitored by sonographic and/or EMG (electromyography) imaging means in this scenario as well.

The present invention is further directed to a pharmaceutical composition and/or to a process for preparation of a pharmaceutical composition according to the present invention. Preferably, said process comprises a step of admixing an expanded population of SMDCs according to the present invention with a pharmaceutically acceptable diluent, excipient, or carrier.

Further, the present invention is directed to a pharmaceutical pack comprising one or more compartments, wherein at least one compartment comprises an expanded population of SMDCs according to the present invention.

The present invention is also directed to the use of an expanded population of SMDCs according to the present invention as a medicament, in particular for preventing and/or treating neuromyopathies, myopathies and/or muscle dysfunctions.

The present invention is further directed to the use of a population of SMDCs according to the present invention in the manufacture of a medicament for preventing and/or treating neuromyopathies, myopathies and/or muscle dysfunctions.

Preferably, the neuromyopathies and/or myopathies as outlined above are or result in incontinence such as fecal incontinence and/or urinary incontinence. Preferably, the muscle dysfunction as outlined above is a skeletal muscle dysfunction such as incontinence, preferably fecal incontinence and/or urinary incontinence.

Finally, the present invention provides a method of treating neuromyopathies, myopathies and/or muscle dysfunctions as defined above comprising administering an expanded population of SMDCs according the present invention to a subject in need thereof.

The subject to be treated or prevented from developing a disease according to any of the above-mentioned embodiments is preferably a human or an animal, in particular a mammal, most preferably a human.

It is contemplated that any method, expanded population of SMDCs or composition described herein can be implemented with respect to any other method, expanded population of SMDCs or composition described herein.

The following examples explain the present invention but are not considered to be limiting.

**Example 1 - Cultivation of SMDCs on Hillex II under static conditions**

To obtain basic information on cell attachment, optimal seeding density, subcultivation techniques, harvesting methods, cell number, and myogenic identity of SMDCs on *Hillex II* microcarriers (Sartorius), several small-scale experiments (6-well plates) were performed and the findings were used for subsequent 3D cultivation in spinner flasks, as described in Example 9.

**1.1 - SMDCs demonstrate high attachment efficiency to microcarriers**

First experiments showed an efficient attachment of SMDCs to *Hillex II* microcarriers, which was further investigated by assessing the number of unattached cells after one hour inoculation with *Hillex II* at different seeding densities of 1,250 / 2,500 / 5,000 and 10,000 cells/cm$^2$. The proportion of unattached but viable cells ranged from 8 to 33%, with the highest seeding densities having the lowest proportions of unattached cells.

**1.2 Assessment of optimal SMDC-to-microcarrier ratio for seeding**

There are three dependent parameters for successful cultivation of SMDCs on microcarriers:

> Number of cells seeded = seeding density; cells per cm$^2$
> Growth surface per volume of growth medium = microcarrier density; cm$^2$ per ml
> Total cell number in culture = cell density; cells per ml

The optimal amount of microcarriers (microcarrier density; cm$^2$ per ml) was assessed by comparing two different densities which were inoculated with different numbers of SMDCs (seeding density; cells per cm$^2$), while the total volume of growth medium remained the same. Microcarrier densities between 5 cm$^2$ to 10 cm$^2$/ml were tested in combination with seeding densities of 1,250 / 2,500 / 5,000 cells/cm$^2$. It was observed that higher seeding densities led to rapid confluence of SMDCs on microcarriers, which impaired growth due to lack of available surface. Comparison of both microcarrier densities of 5 and 10 cm$^2$/ml demonstrated that a higher microcarrier density resulted in increased SMDCs doubling time, an effect which is explained by overall higher cell density (cells per ml). This suggested that a lower microcarrier density of 5 cm$^2$/ml was beneficial for SMDCs expansion at the tested seeding densities. To assess the optimal seeding density at 5 cm$^2$/ml microcarrier density, three independent cell batches were cultured on microcarriers at different seeding densities for four to 15 days, and the doubling times were compared (Table 1). In summary, doubling time of SMDCs increased proportionally with seeding density (cells per cm$^2$) without exception, likely due to less medium per cell being available as the total cell number in culture increased. Notably, cells were able to grow at even very low seeding densities of 500 cells/cm$^2$ under static conditions, indicating that cell density (cells per ml) is an important parameter, given that enough surface area for growth was provided. Flow cytometry measurement showed that there were no considerable differences in CD56 expression between higher and lower cell seeding densities, suggesting a robust culture system for SMDCs expansion.

*Table 1 - Influence of seeding density on SMDCs doubling time.*

| Seeding density [cells/cm2] | Cultivation time [days] | Doubling time [days] |
|---|---|---|
| 2,500 | 4 | 1.29 |
| 5,000 | 4 | 1.32 |
| 500 | 15 | 2.60 |
| 1,000 | 15 | 2.81 |
| 1,500 | 15 | 3.55 |
| 2,000 | 15 | 3.60 |
| 2,500 | 15 | 4.13 |
| 2,000 | 7 | 1.75 |
| 4,000 | 7 | 2.27 |
| 6,000 | 7 | 2.63 |
| 8,000 | 7 | 3.50 |
| 10,000 | 7 | 3.63 |

Microcarrier density of 5 cm$^2$/ml was used.

According to these findings, it is recommended to choose a ratio with low seeding density and high medium volume with enough surface area (microcarriers) for expansion of SMDC.

**1.3 - Bead-to-bead transfer for cell expansion on microcarriers**

Splitting is an essential step during cell culture to enable continuous cell growth by providing sufficient growth surface. Three methods for splitting were compared using SMDCs grown on *Hillex II* microcarriers:

- Enzymatic detachment of cells from microcarriers, used microcarriers were discarded, and re-seeding on new microcarriers (analogous to 2D culture splitting).
- Enzymatic detachment of cells from microcarriers, re-seeding on used and new microcarriers (mix of old and new microcarriers without separation).
- Addition of new microcarriers to culture, without enzymatic detachment of cells (termed *bead-to-bead-transfer*).

As control, SMDCs were cultivated on microcarriers without subculturing. No significant differences regarding microcarrier colonization and CD56 expression were observed between all tested methods.

When SMDCs on microcarriers where confluent, indicated by agglomeration of microcarriers and dense cell population on the majority of microcarriers, cells were harvested, counted and CD56 expression was measured. Bead-to-bead transfer led to comparable results among the tested conditions. This demonstrates that expanded cells are able to attach to new microcarriers during cultivation.

Moreover, it was tested whether the addition of new Hillex II microcarriers after day 3, 4, 5, 6 or 7 has an influence of CD56 expression of the resulting SMDCs. These tests were compared with no addition of Hillex II microcarriers. The total cultivation time was each nine days. Syto24 staining of cell nuclei revealed comparable population of the microcarriers. No differences in CD56 expression were detected (no addition: 96.7 % CD56 expressing SMDCs; addition after 3 days: 97.1 % CD56 expressing SMDCs; addition after 4 days: 96.8 % CD56 expressing SMDCs; addition after 5 days: 97.3 % CD56 expressing SMDCs; addition after 6 days: 97.4 % CD56 expressing SMDCs; addition after 7 days: 97.1 % CD56 expressing SMDCs).

Notably, the risk of premature fusion increases with higher confluency levels, thus sufficient surface area for growth must be provided and agglomeration of microcarriers during SMDCs expansion should be avoided.

**Example 2 - Harvest of SMDCs on microcarriers**

To harvest cells from microcarrier culture, medium was removed and microcarriers with cells were washed twice with 1x DPBS (-Ca$^{2+}$/-Mg$^{2+}$). Cells were detached from microcarriers with either Trypsin or *TrypLE select* enzyme (Thermo Fisher Scientific Inc., MA, USA) and separated from the microcarriers by passing the microcarrier-cell suspension through cell strainers (40-100μm). The obtained single cell suspension was then used for downstream applications, cryopreservation, or analysis according to standard procedures.

**Example 3 - Syto24 staining of cells on microcarriers**

To assess the population density on microcarriers, staining of cell nuclei was performed by transferring 300-1000 μl microcarrier-cell suspension to a 24-well plate and adding Syto™24 Green Fluorescent Nucleic Acid Stain (Thermo Fisher Scientific Inc., MA, USA) to a final concentration of 0.5 μM. After incubation for 15-60 min at 37°C, pictures were acquired with Incucyte S3 (10x objective, phase contrast, and green channel with 150 ms exposure time).

**Example 4 - Cell count**

Cell count was determined with Nucleocounter after detachment and separation from microcarriers as described in Example 2. For some experiments microcarrier-cell suspension was directly tested for counting without prior harvesting procedure (as indicated).

**Example 5 - Flow cytometry**

Flow cytometry analysis was performed on a Guava easyCyte 6HT 2L flow cytometer (Merck Millipore, Darmstadt, Germany). Briefly, cells were harvested as explained in Example 2 and resuspended in growth medium. Cells in a concentration of 40000/reaction were incubated with 5 to 10 μL anti-CD56-PE (Beckman Coulter Inc., France), anti-CD90-PE (Beckman Coulter Inc., France), anti-CD34-PE (Beckman Coulter Inc., France), and Isotype IgG1-PE (Beckman Coulter), in a 96-well round bottom plate, and each reaction received 5 μL of viability dye 7-aminoactinomycin D (Beckman Coulter Inc., France). The samples were incubated for 20 min at room temperature in the dark. In addition, cell cycle analysis was performed by incubating cells with Vybrant™ DyeCycle™ Green Stain (Molecular Probes™, Thermo Fisher Scientific Inc., MA, USA) for 30 min at 37°C. Cell events were acquired with the help of Guava InCyte™ v.3.3 software. Histograms and dot-plots were generated with a minimum of 5000 events with a sample flow rate of 0.6 μL/s. Positive staining was obtained by comparison with Isotype control set as at least 99% negative.

## Example 6 - Immunostaining of myosin heavy chain and smooth muscle

Cells were seeded in a gelatine coated 24-well plate ($1 \times 10^5$ cells per well) and incubated at 37°C and 5% $CO_2$ in growth medium. After 24 to 48 hours cells were fixed, and immunostaining was performed. For fixation, culture medium was removed, and the wells were washed three times with 1 ml 1x DPBS ($-Ca^{2+}/-Mg^{2+}$), then 400 μl 4% paraformaldehyde (PFA) was added for ten minutes at room temperature under a digestor cabinet. After wells were washed three times with DPBS-T (1x DPBS with 0.1% Triton-X-100), primary antibody incubation was started. Alternatively, cells were stored prior to permeabilization with DPBS-T in 1x DPBS at 4°C until staining. Primary antibodies (mouse anti-myosin heavy chain / MHC, or mouse anti-smooth muscle actin / SMA, or rabbit anti-Desmin) were diluted 1:300 in blocking solution (1x DPBS containing 3% goat serum and 0.1% Triton-X-100) with 0.5 μM Syto24. 300 μl of primary antibody + Syto24 solution were added to each well and incubated for 90 to 120 minutes at 37°C. Next, wells were washed three times with DPBS-T. The secondary antibody (goat anti-mouse Alexa Fluor 647, or goat anti-rabbit Alexa Fluor 647) was diluted 1:500 in blocking solution, and 300 μl antibody solution was added per well and incubated for 60 to 90 minutes at 37°C in the dark. Secondary antibody solution was removed, and wells washed three times with DPBS-T, and 1x DPBS was added to the wells for imaging. Imaging was performed with Incucyte S3 imaging system (Sartorius AG, Göttingen, Deutschland) with the following settings: 10x objective, phase contrast, green (150 ms exposure time) and red (600 ms exposure time) channel. Images were analysed with the Incucyte software with the analysis tool, and brightness, contrast and colour intensity were adjusted individually for each measurement.

## Example 7 - AChE potency and Myogenic Potency of SMDC

To measure Acetylcholinesterase (AChE) activity, 200,000 SMDCs are seeded in a gelatin-coated 24-well plate. Cells are induced to differentiate and fuse to form multinucleated myotubes using SMDCs differentiation medium for five to seven days. After this period, the differentiation medium is carefully removed and immediately replaced with 300 μL of 0.5 mM 5,5'-dithiobis-2-nitrobenzoic acid (DTNB) solution (prepared in phosphate buffer, pH 7.2, with 0.1% Triton X-100). Following this, 50 μL of 5.76 mM Acetylthiocholine iodide (ATI) (prepared in distilled water) is added. The optical density (OD) is measured at 412 nm using a Spark microplate reader (Tecan Trading AG, Männedorf, Switzerland). A blank reaction, which includes all reagents except cells and gelatin coating, is performed in two separate wells. The corrected OD412nm values are determined by subtracting the mean OD412nm of the blank wells from the mean OD412nm of the sample wells. The AChE activity in relative milliunits (AChE mUrel) is calculated using the equation from a standard curve. The standard curve is generated by measuring dilutions ranging from 4 to 500 mU/mL of a ready-to-use 50 U/mL AChE stock (from Electrophorus electricus, AAT Bioquest® Inc., Sunnyvale, CA, USA). The standard dilutions are prepared in phosphate buffer (pH 7.2 with 0.1% Triton X-100) and used immediately. For the AChE standard enzyme analysis, 200 μL of each dilution is mixed with 300 μL of 0.5 mM DTNB and 50 μL of 5.76 mM ATI, and the OD412nm is measured after 60 minutes in a 24-well plate.

SMDCs that are of myogenic lineage are able to fuse and form syncytial myotubes in vitro that are representative for skeletal muscle fibers in vivo. Thus, myogenic potency of the SMDCs can be tested by in vitro differentiation. Cells to be tested were induced to differentiate as described above. SMDCs formed huge myotubes that were Myosin Heavy Chain (MHC) positive. Respective results confirm the myogenic potency of SMDCs which is necessary to functionally regenerate skeletal muscle tissue.

## Example 8 - Myogenic identity of SMDCs grown on microcarriers

In addition to cell growth, ensuring maintenance of defined quality parameters related to myogenic identity is crucial for generating a comparable and safe product. Thus, the impact of cultivating SMDCs on *Hillex II* microcarriers on the percentage of CD56 positive cells, their fusion capability upon harvest as well as potential premature fusion during expansion, and unwanted lineage conversion towards smooth muscle-like cells were investigated.

## 8.1 - SMDCs grown on microcarriers demonstrate stable or increased expression of CD56

The influence of SMDCs expansion on *Hillex II* microcarriers on the ratio of myogenic to non-myogenic cells over time was examined by determining the percentage of CD56⁺ cells by flow cytometry before and after culture on microcarriers under static conditions. In all experiments (n = 6) the proportion of the myogenic CD56$^+$ population was at least stable ($\pm$ 1%) or slightly increased upon three to nine days of cultivation on *Hillex II* microcarriers, as compared to the starting population and after parallel cultivation in standard 2D cell culture, as shown in exemplary in Fig. 1. These results suggest that growth on Hillex II microcarriers is beneficial for myogenic cells.

### 8.2 - No premature fusion nor lineage conversion in SMDCs grown on microcarriers

Immunostaining of MHC and SMA was performed on cells after harvest of SMDCs grown on *Hillex II* microcarriers to exclude potential premature fusion or differentiation towards a smooth muscle-like phenotype. They were compared to standard cultivation in flasks (2D). There were no signs of premature fusion in both culture conditions, as shown by lack of MHC signal. While a weak SMA signal in few cells was observed in 2D cultured SMDC, the signal was further decreased in 3D cultured SMDC. Images were acquired with IncuCyte S3 imaging system. Nuclei were stained with Syto24 dye (green). Thus, neither MHC nor SMA were detected in SMDCs after cultivation on *Hillex II* microcarriers under static conditions, indicating that SMDCs maintained their myogenic progenitor identity over the duration of expansion.

### Example 9 - Cultivation of SMDCs on Hillex II under stirred conditions in spinner flasks

With static condition experiments, it was shown that cultivation of SMDCs on *Hillex II* microcarriers was possible while quality parameters of myogenic identity were maintained, and several basic aspects for cultivation of SMDCs on *Hillex II* microcarriers were clarified. Next, SMDCs culture on *Hillex II* microcarriers was transferred to a stirred setup, ultimately aiming at GMP compliant production of CD56 expressing SMDCs in bioreactors. A stirred setup by transferring SMDC-microcarrier cultures to spinner flasks was developed to i) determine the required conditions for a stirred system, and ii) assess whether quality parameters were still similar to previous cultivation.

### 9.1 Stirring speed determines SMDCs attachment efficiency to microcarriers

The manufacturer's user guide for *Hillex II* microcarriers as well as recent literature recommend a static phase upon cell seeding on microcarriers for facilitating attachment. As it had already been shown in static condition that the majority of SMDCs attached to microcarriers within one hour, the same timespan was adopted for spinner flasks. Thus, a static attachment phase of one hour was allowed for these experiments. After the attachment phase, the stirring phase started. However, as the cultured cells are able to attach to the microcarriers during stirred cultivation, the static attachment phase can also be omitted or performed at a very low stirring speed of about 10 to 30 rpm.

Especially the stirring speed was an importantcm2/ml parameter to define as it should be high enough to keep all microcarriers floating but as low as possible to minimize shear stress. First tests in 125 ml single-use spinner flasks with 30 ml medium and a stirring speed of 30 rpm failed as microcarriers could not be kept in suspension permanently and big agglomerates formed at the bottom in the centre. Thus, the medium volume was increased to 60 ml to ensure complete immersion of the paddle while the stirring speed was increased to 45 rpm. After these adaptions, microcarriers were floating more homogenously and only small clumps were forming. Notably, with higher confluences more agglomerates started to form which were not kept in suspension with stirring at 45 rpm. As no negative impact on SMDCs growth or quality parameters was found and additional shear stress was avoided, stirring speed was not further increased at first and microcarrier agglomeration was used as an indicator for increasing cell density.

### 9.2 - Microcarrier-to-medium ratio determines SMDCs growth

Seeding densities between 3 x10$^3$ and 1.7 x10$^4$ cells/cm$^2$ were tested, all of which allowed cultivation of SMDCs on *Hillex II* microcarriers in spinner flasks while maintaining high CD56 expression levels between 83% to 99% (when populations with at least 80% CD56$^+$ cells were used as starting material). This demonstrated the robustness of the system within a wide seeding range for SMDCs cultivation yet again.

Microcarrier density was another important factor which must be considered, thus densities between 2.5 and 17 cm$^2$/ml were tested and SMDCs growth compared (see Fig. 2A). At microcarrier densities above 10 cm$^2$/ml cells were initially able to attach but were not able to grow sufficiently, ultimately resulting in cell detachment and death after several days. In contrast, microcarrier densities between 2.5 and 7.2 cm$^2$/ml resulted in stable growth of SMDC.

Besides seeding density (cells per cm$^2$) and microcarrier density (cm$^2$ per ml) also the ratio of medium volume to cells (cell density, cells per ml) was relevant. Comparison of seeding densities between 1.6 x10$^4$ to 5 x10$^4$ cells/ml revealed that growth rate was positively correlated with volume of medium per cell, as shown in (see Fig. 2B)

Taken together, while the seeding density of SMDCs on microcarriers was rather flexible, the microcarrier density should be below 7.2 cm$^2$/ml and the medium volume high to achieve stable growth and survival of SMDCs on microcarriers in the stirred spinner flask setup.

### 9.3 - Bead-to-bead transfer is feasible in spinner flask system

As bead-to-bead transfer was chosen as the best option for splitting in static settings, this method was also tested for cultivation in spinner flasks. Cells were cultivated until microcarriers were confluent and started to agglomerate, then new microcarriers were added. For better initial attachment upon addition of new microcarriers, a static phase of one hour was implemented. Theoretically, it could be more difficult for SMDCs to populate new microcarriers during expansion while being in motion within the stirred spinner flask. However, bead-to-bead transfer in the stirred setup was successful in all tests, as new microcarriers were uniformly populated within a few days. It was observed that while many unpopulated microcarriers were present directly after addition, most microcarriers were populated with at least some cells within one day. After three days, cells were uniformly distributed on all available microcarriers.

### 9.4 - Assessment of cell number and confluence in spinner flask system

SMDCs growth was estimated based on the size of microcarrier agglomerates, total cultivation days and estimation of microcarrier population as assessed by Syto24 staining.

### 9.5 - Comparable SMDCs doubling rate in 3D and standard 2D culture

SMDCs in standard 2D culture grow at a doubling rate of approximately two days. Doubling rate was calculated as follows (t = time in culture, N end = harvest cell number, N start = starting cell number):

$$t_d = \text{t} \cdot \frac{\ln 2}{\ln \left( \frac{N_{end}}{N_{start}} \right)}$$

Growth rates in spinner flasks displayed wide variations ranging from 1.3 days up to around five days depending on i) medium volume, ii) microcarrier density and iii) the cells used in the respective experiment. When SMDCs were seeded in low densities of approximately $1.6 \times 10^4$ cells/ml and cultivated for a maximum of seven days, doubling times between 1.3 to 2.5 days were comparable to standard 2D cultivation. When higher cell concentrations were used or when cells were cultivated for more than seven days, doubling times increased considerably to up to 4.9 days, likely due to insufficient surface area for growth and nutrient supply (medium volume too low for cell density).

Additionally, cell cycle analysis was used to compare the proportion of dividing cells in cell populations following either cultivation on *Hillex II* microcarriers in spinner flasks or after 2D culture in cell culture flasks. The results demonstrated similar values for both conditions with 8-20% dividing cells during cultivation in spinner flask and 11-14% during 2D cultivation which is consistent with comparable growth rates.

While it is essential to optimize the parameters of cell density (cells per ml) and microcarrier density ($cm^2$ per ml) to maintain cells at a comparable growth rate until harvest, the experiments showed that SMDCs obtained from cultivation on *Hillex II* microcarriers under stirred conditions in spinner flasks showed a similar growth rate as SMDCs from standard 2D culture. Most importantly, the ratio of proliferative (= mitotically active) to non-proliferative (= mitotically inactive) cells of 3D cultured SMDCs is highly similar to standard 2D cultured SMDC, suggesting an efficient and uniform expansion process.

### 9.6 - Stable or increased CD56 expression in spinner flask SMDCs

As already suggested by experiments in static condition, the percentage of CD56+ SMDCs increased upon cultivation on *Hillex II* microcarriers in spinner flasks in the vast majority of experiments or remained stable at around 95 ±5% (n= 25 out of 28). Surprisingly, the CD56+ population increased by up to 59% when mixed populations (>30% CD56-) were used as starting material, clearly indicating a beneficial culture environment for myogenic SMDCs (Fig. 3).

To determine whether the growth conditions on microcarriers in spinner flasks are suboptimal for non-myogenic SMDC, a 0.4% CD56+ SMDCs population was cultured in the 3D setup for seven days and measured. Notably, the results showed a comparable doubling time of 2.4 days in absence of a dominant CD56+ SMDCs population (0.8% CD56+ at endpoint analysis). Thus, the observed increase of myogenic CD56+ SMDCs in mixed cultures when propagated in the novel system was not due to impaired growth of non-myogenic CD56- SMDCs in general. In conclusion, myogenic SMDCs benefit from cultivation on *Hillex II* microcarriers in spinner flasks according to CD56 key marker expression. While non-myogenic CD56- cells have a known growth advantage in standard 2D culture, the novel 3D system was shown to reverse this advantage in favor of the myogenic CD56+ population.

**Example 10 - Biopsy processing, digestion and start of primary culture**

Biopsies were processed as described in Example 1 of WO 2019/115790 with slight variations. Briefly, biopsy samples were processed, subsequently cooled at 2 to 8°C for 48 hours and digested for 20 hours at 37°C and 5% $CO_2$ with collagenase (Ham's F10 + HEPES Buffer + Gentamycin + 10% FCS + 0.14% Collagenase). Primary culture was started in standard growth medium as described in Example 1 of WO 2019/115790 and plated on uncoated cell culture flasks with the modification that gentamycin was added. First medium change of primary culture was performed after four to 24 hours following seeding. Afterwards, medium was changed every two to three days.

**Example 11 - Preparation of and cell seeding on Hillex II microcarriers**

Stock solutions of 50 $cm^2$/ml of *Hillex II* microcarriers in autoclaved $dH_2O$ were prepared according to manufacturer's instructions and stored at room temperature.

Before seeding cells onto microcarriers, microcarriers were acclimated to the medium. The desired amount of microcarrier stock solution (600 $cm^2$) was transferred into a spinner flask and $dH_2O$ was aspirated after microcarriers had settled. Afterwards, microcarriers were incubated in growth medium at 37°C for at least 30 minutes prior to seeding. Following acclimation of microcarriers in culture medium, single cell suspension of SMDCs in growth medium was added, and gentle movements of the spinner flasks ensured homogenous cell and microcarrier distribution within the vessel. Cells were incubated statically for two hours at 37°C and 5% $CO_2$ after seeding to facilitate attachment prior to stirring.

**Example 12 - Cultivation of SMDCs on microcarriers**

Cells on *Hillex II* microcarriers were cultivated in standard SMDCs growth medium (Hams F10, 10% FCS, growth factors such as bFGF and Gentamicin). 500 ml single-use spinner flasks were used to seed and incubate cells in 250 ml total volume at 37°C and 5% $CO_2$. Spinner flasks were placed on a magnetic stirrer and a suitable stirring speed at which microcarriers were floating was chosen, which was usually between 30 and 45 revolutions per minute (rpm). Medium change was performed every second day by stop of stirring, letting SMDCs on microcarriers settle down, removing half of the culture medium (125 ml) from the vessel and replacing it with the same volume of fresh medium. In total, between 790 - 850 ml of growth media were used for expansion to ≥50 million SMDCs by 3D cultivation. Control cells were cultivated in T25, T75 or T175 flasks according to the 2D standard procedure.

**Example 13 - Bead-to-bead transfer for SMDCs expansion**

For subculturing of SMDCs on microcarriers, the desired volume of *Hillex II* stock solution (addition of 600 $cm^2$) was transferred to a Falcon tube, and after microcarriers had settled down, the supernatant was carefully aspirated. Microcarriers were acclimated with culture medium for at least 30 minutes at 37°C and 5% $CO_2$, and afterwards added to the spinner flask culture. Stirring was paused for one hour to facilitate attachment of cells to new microcarriers and resumed afterwards. Successful bead-to bead transfer onto the newly added microcarrier was observed.

**Example 14 - Harvest of SMDCs on microcarriers**

Medium was removed and microcarriers with cells were washed twice with 1x DPBS (-$Ca^{2+}$/- $Mg^{2+}$). At harvest, a total of 1200 $cm^2$ of microcarriers were in culture (600 $cm^2$ initial seeding + 600 $cm^2$ for subculture). Cells were detached from microcarriers with *TrypLE select* enzyme and separated from the microcarriers by passing the microcarrier-cell suspension through a cell strainer series (15 μm cells strainer stocked beneath a 40 μm cell strainer). A funnel was used for easier handling and a connector ring was used to connect vacuum pump to facilitate filtration. Single cell suspension containing SMDCs was collected in falcon tubes for subsequent centrifugation and downstream applications (e.g. analysis, cryopreservation).

**Example 15 - Density estimation and cell count of SMDCs on microcarriers, flow cytometry and AChE potency assay**

To assess the population density on microcarriers, Syto24 staining of cell nuclei was performed by transferring 300-1000 μl microcarrier-cell suspension to a 24-well plate and adding Syto24 dye to a final concentration of 0.5 μM. After incubation for 15-60 min at 37°C, images were acquired with Incucyte S3 (10x objective, phase contrast and green channel with 150 ms exposure time). Cell count was determined with Nucleocounter after detachment and separation from microcarriers. For some experiments microcarrier-cell suspension was directly tested for counting.

Flow cytometry analysis to assess viability via 7AAD nucleic dye and expression of CD56 and CD90 was performed as described above in Example 5.

AChE potency assay was performed as described above in Example 7. The usual differentiation time of five to seven days was shortened to three days due to extensive fusion and to avoid tube detachment.

**Example 16 - Successful expansion of SMDCs to over 50 million**

SMDCs cultured according to the novel 3D culture setup using *Hillex II* microcarriers and spinner flasks enabled expansion of at least 50 million cells successfully and reliably. An overview of the three individual batches is shown in Table 2. SMDCs demonstrated typical myoblast morphology following expansion in 3D as compared to standard 2D.

*Table 2. Overview of SMDCs real-scale 3D expansion tests.*

| Biopsy | Days in primary culture | Primary culture output* [mio] | Days in spinner flask | Doubling time [days] [1] | Harvest [mio cells] |
|---|---|---|---|---|---|
| SMDC230131-1 | 13 | 6.4 | 9 | 2.6 | 68 |
| SMDC230131-2 | 11 | 1.8 | 11 | 1.9 | 90 |
| SMDC230131-3 | 12 | 5.0 | 10 | 2.4 | 95 |

* Cells harvested from primary culture were seeded for spinner flask system (minus cells used for flow cytometry analysis).

[1] Average doubling time for SMDCs obtained from standard 2D culture is two days.

**Example 17 - High expression of CD56 following 3D expansion**

Following cultivation in the 3D culture setup for nine to 11 days and expansion to $\geq$ 50 million cells, SMDCs showed high expression of $CD56^+$ myogenic cells between 99.2% (SMDC230131-1), 98.9% (SMDC230131-2), and 97.8% (SMDC230131-3), as shown in Fig. 4. This demonstrates the robustness of the system to obtain a highly pure target cell population for medical applications.

**Example 18 - Successful myotube fusion of SMDCs following 3D expansion**

SMDCs were able to fuse and form multinucleated, millimeter-sized myotubes Fig. (5A) in combination with acetylcholinesterase activity of 211, 286 and 308 mUrel/ml, respectively (AChE, Fig. 5B). This indicates that SMDCs obtained from 3D cultivation were functional in terms of basic skeletal muscle physiology.

**Example 19 - Cultivation of SMDCs on microcarriers in DASbox® bioreactor system**

For this example, three cell batches of 70% $CD56^+$ mixed population SMDCs (termed "Batch 1.2", "Batch 2", and "Batch 3") were used, and standard adherent monolayer culture was compared to cultivation in the DASbox® bioreactor system (Eppendorf SE) in terms of CD56 expression dynamic and population growth over the course of cell expansion.

After recovery of the cells according to Example 10 from cryopreservation, they were detached from TC-treated T175 flasks and seeded onto Hillex® II microcarriers (Sartorius AG) in a BioBLU® 0.3 sc bioreactor vessel (Eppendorf SE). Microcarrier preparation and seeding procedure was performed as described in detail in Example 11. Following seeding, the bioreactor vessel was placed into the bioreactor system, where cells were incubated at 37°C and were supplied with 5% $CO_2$. After a static phase of 90 minutes to facilitate initial cell attachment onto microcarriers, continuous stirring at 90 rpm (counterclockwise) was applied throughout further cultivation.

Automated medium change (under continuous stirring) was set to exchange i) 40 ml every 24 hours from day 1 to 2, ii) 40 ml every 12 hours from day 3 and 4, and iii) 40 ml every 8 hours from day 5 on, accounting for higher medium consumption by an increasing SMDCs population density. Table 3 gives an overview of the cultivated batches and setup.

*Table 3: Parameters for cultivation of SMDCs batches in* DASbox® *bioreactor system.*

| Cell batch | Batch 1.2 | Batch 2 | Batch 3 |
|---|---|---|---|
| Amount of Hillex II microcarriers | 1200 cm$^2$ | 1200 cm$^2$ | 1200 cm$^2$ |
| Cells seeded | $3.5 \times 10^6$ | $3.5 \times 10^6$ | $2.4 \times 10^6$ |
| Medium volume within bioreactor | 250 ml | 250 ml | 250 ml |
| Total medium consumption | 730 ml | 730 ml | 970 ml |

(continued)

| Cell batch | Batch 1.2 | Batch 2 | Batch 3 |
|---|---|---|---|
| Days of cultivation in DASbox® | 7 | 7 | 9 |

Harvest procedure was performed manually by disconnecting the bioreactor vessel from the bioreactor instrument and detaching SMDCs from microcarriers enzymatically as described in Example 14. Decision for timepoint of harvest was based on estimated confluence of SMDCs on microcarriers as previously described. Flow cytometry to assess viability and expression of CD56 as well as AChE potency assay to determine fusion capability was performed as described in Example 15. In comparison to Example 13, no bead-to-bead transfer was performed by adding additional microcarriers. Instead, the total amount of microcarriers was added at the beginning as described above and outlined in Table 3.

Cultivation on Hillex® II microcarriers within the DASbox® bioreactor system as described above resulted in robust SMDCs expansion to at least $50 \pm 10$ million cells. The observed doubling times ranged from 1.6 to 2.2 days, remaining within the typical range observed during the cultivation of SMDCs as per the standard manufacturing process. An overview of the cultivation of the three individual batches is shown in Table 4.

*Table 4: Overview of SMDCs growth in DASbox® bioreactor system.*

| SMDCs batch | Seeded in bioreactor [mio cells] | Days in bioreactor | Doubling time [days] | Doubling time [days] in 2D | **Harvest from bioreactor [mio cells]** |
|---|---|---|---|---|---|
| Batch 1.2 | 3.5 | 7 | 1.6 | 1.4[1] | **69.6** |
| Batch 2 | 3.5 | 7 | 1.7 | 1.3[1] | **63.0** |
| Batch 3 | 2.4 | 9 | 2.2 | 2.4[1] | **44.0** |
| [1] Average doubling time during standard cultivation as adherent monolayer. | | | | | |

Following cultivation in the DASbox® bioreactor system for seven to nine days and expansion to 44 to 70 million cells, percentage of CD56+ myogenic cells increased in all SMDCs populations by 8 to 26 percent, while CD56 expression decreased drastically in all experiments that were performed with the same three batches in standard adherent monolayer (2D) culture (within three passages, 13 to 20 days total cultivation time) as shown in Table 5.

*Table 5: Overview of results of flow cytometry analysis of CD56 expression. Percentage of CD56+ myogenic cells increased during cultivation in DASbox® bioreactor system whereas it decreased during standard cultivation as adherent monolayer. In both culture conditions, standard FCS-based growth medium was used.*

| SMDCs batch | Proportion of CD56+ cells prior cultivation | Proportion of CD56+ cells after cultivation | Increase (+)/Decrease (-) of proportion of CD56+ cells during cultivation |
|---|---|---|---|
| Batch 1.2 standard | 72% | 48% | -24% |
| Batch 2 standard | 77% | 25% | -52% |
| Batch 3 standard | 67% | 50% | -17% |
| Batch 1.2 bioreactor | 74% | 86% | +14% |
| Batch 2 bioreactor | 79% | 87% | +8% |
| Batch 3 bioreactor | 56% | 82% | +26% |

SMDCs of all three batches were able to fuse and form multinucleated myotubes with at least three nuclei per myotube in combination with acetylcholinesterase activity (AChE). This indicates that SMDCs obtained after expansion in DASbox® bioreactor system were functional in terms of basic skeletal muscle physiology.

As outlined above, the feasibility of the automated expansion of SMDCs on Hillex® II microcarriers in the DASbox® bioreactor system was demonstrated. Notably, the cells exhibited a sustained and stable growth pattern, affirming the general capacity of the DASbox® configuration to support the cultivation of more than 50 million myogenic SMDC. Furthermore, a consistent enrichment of CD56+ cells within the SMDCs population was observed across all three batches, enabling generation of a highly myogenic population for final formulation. Beyond increased expression of CD56, bioreactor derived SMDCs demonstrated their fusion capability by forming multinucleated myotubes upon differentiation. The observed enrichment of CD56$^+$ cells through 3D cultivation on Hillex® II microcarriers constitutes a substantial advantage when compared to the conventional 2D cultivation process which is often marked by a progressive decrease of myogenic cells throughout the propagation period.

In conclusion, SMDCs which were cultured on Hillex® II microcarriers in the DASbox® bioreactor system demonstrated robust growth and met all established quality control criteria, including myogenic identity (CD56) and fusion ability (AChE). Notably, not only stabilization but enrichment of CD56$^+$ expressing cells within the heterogenous SMDCs population was achieved, representing a significant milestone for future manufacturing of SMDCs. Consequently, the cultivation of SMDCs in the DASbox® bioreactor not only signifies a substantial advancement towards the automation of the manufacturing process but also holds promise for enhancing the overall product quality.

## Example 20 - Comparison of 1-step digest and 2-step digest

For optimizing the digestion step after biopsy processing as described in Example 10, a 2-step digestion was tested and compared with the results of a 1-step digestion as described in Example 10. The first step of said 2-step digestion corresponds to the digestion as described in Example 10 performed for 20 hours at 37°C, 5% $CO_2$ in collagenase. For performing the second step, the sample obtained after collagenase digestion was pelleted by centrifugation at 400g (rcf) for 10 minutes at room temperature, the supernatant was aspirated and the pellet was resuspended in a digestion solution comprising Trypsin (NaCl-Lösung (Natrium-Chlorid) + 1X Trypsin-EDTA) and digested for 10 minutes at 37°C, 5% $CO_2$. Afterwards, the tissue digest of both the 1-step digestion and the 2-step digestion were passed through a series of cell strainers (with 100, 40, and 15$\mu$m mesh size). The obtained cells were each seeded in growth medium (10%FCS+bFGF).

Results (Fig. 7): Following digest with Collagenase, skeletal muscle tissue obtained from biopsy gives rise to single cells and fragments of muscle tissue, containing large amounts of cells which are lost during change of the growth medium. A second digest step with trypsin for 10 minutes at 37°C resulted in that only minor fragments remained and more single cells were obtained. It was further observed that after the tissue digest was passed through a series of cell strainers (e.g., 100 - 40 - 15$\mu$m mesh size) to remove all remaining fragments, a higher amount of cells was obtained when performing the 2-step digest in comparison to the 1-step digest.

## Example 21 - Effect of Biolaminin-coating on myoblast growth and CD56 expression in preliminary 2D culture

Biopsies obtained from pectoral muscle were processed according to standard procedure as described herein. After primary culture, it was observed that the amount of the CD56 positive population declined continuously. Therefore, it was tested whether Biolaminin-coating can slow down the decrease of CD56 positive cells, or maybe even stabilize the CD56$^+$ population over several splitting steps. It was also examined whether use of flasks with CellBIND surface in addition to Biolaminin-coating can further improve the performance.

Cells after primary culture were either grown in standard cell culture T75 flasks, Biolaminin-coated cell culture T75 flasks, or Biolaminin-coated T75 flasks with CellBIND surface. Cells were cultivated for two passages, whereas every time cell number was adjusted. At every split and at harvest the percentage of CD56 positive cells was determined by flow cytometry.

### 21.1 Biolaminin-coating

Biolaminin 521 LN (research-grade, Biolamina) was used. Prior to use, Biolaminin was diluted to a working concentration of 5 ng/ml in PBS with Ca2+/Mg2+. T75 flasks were coated with 8 ml Biolaminin solution each, for either 2 h at 37°C or 24 h at 4°C. Shortly before cells were seeded into flasks, Biolaminin solution was removed from flasks.

### 21.2 Cell culture setup and vessels

Cells were used after primary culture. 625.000 cells were seeded i) in an uncoated T75 flask (Sarstedt), ii) a Biolaminin-coated T75 flask (Sarstedt), and a iii) Biolaminin-coated CellBIND T75 flask (Corning). Medium change was performed every 2-3 days, and cells were split when ~70-80% confluent. After the first and second split, 500.000 cells were seeded in each T75 flask, and remaining cells were used for flow cytometry measurement and frozen for future experiments. Splitting and harvesting was performed according to standard procedure.

### 21.3 Flow cytometry

Flow cytometry was performed according to standard procedure by using 5 μl of antibody (IgG-PE, CD56-PE and CD90-PE) and 195 μl cell suspension (200.000 cells/ml) followed by an incubation time of 20 min.

### 21.4 Results (Fig. 8)

Biolaminin-coating considerably increased growth rate compared to cells which were grown on the uncoated cell culture flask, while no differences between Biolaminin-coated cell culture flasks and Biolaminin-coated CellBIND flasks could be detected. Mean doubling time of cells on Biolaminin coating (regardless of CellBIND surface) was 1.1 days, whereas cells on uncoated flasks needed 2 days on average to double.

In uncoated flasks, the percentage of CD56 positive cells considerably dropped from 95% to 35% during cultivation. In contrast, with Biolaminin-coating the percentage of CD56 positive cells remained stable at 94-96% during cultivation. Again, no significant differences between Biolaminin-coated CellBIND and Biolaminin-coated standard cell culture flasks were observed.

### Example 22 - Microcarrier test in DASbox (Eppendorf) comparing distinct microcarriers

Overview of tested microcarriers is shown in the following Table:

| Name | Manufacturer | Size [μm] | Surface area [cm$^2$/g] | Material | Catalog No. |
|---|---|---|---|---|---|
| *Hillex II* | Sartorius | 160-200 | 515 | Polystyrene with cationic amine-modified microporous surface | H-170-020 |
| *Synthemax II* | Corning | 125-212 | 360 | Polystyrene with Synthemax® coating | 3781 |

Human SMDCs (98% CD56$^+$, passage 2) were obtained as described above in Ex. 10. 4 million SMDCs were seeded on 1200 cm$^2$ microcarriers and cultivated as described above except for the mixing: For preventing aggregation of cells/microcarriers, the suspension was mixed as described before at 90 rpm. In addition, starting on day 2 of culture 1-3 times a day the mixing speed was increased for 1min to 140 rpm. The cultivation was performed in a DASbox bioreactor (Eppendorf) for a total of 7 days. Results are shown in the Table 6 and in Fig. 9:

Table 6: Comparison of SMDC expansion using Hillex II and Synthemax II microcarriers

| | Hillex II | Synthemax II |
|---|---|---|
| Harvest | 107 million cells | 34 million cells |
| Doubling time | 1.5 days | 2.3 days |
| CD56 expression | 98% CD56$^+$ (+0%) | 91% CD56$^+$ (-7%) |
| AChE potency | 201 mUrel/200,000 cells | 161 mUrel/200,000 cells |
| Formation of myotubes | + and more defined in comparison to Synthemax II | + |

Better growth was observed on Hillex II microcarriers. It was further observed that more cells attached to the Hillex II microcarriers than on the Synthemax II microcarriers, as more cells stayed in suspension when cultured with Syntemax II microcarriers. Thus, cells in suspension were lost during the harvest procedure as only cells attached to microcarriers were further analyzed. A minor decrease in CD56 expression was observed with Synthemax II microcarriers, while the proportion of CD56 expressing cells was stable with Hillex II. The formation of myotubes was observed after 5 days of SMDCs differentiation. Both SMDCs cultured on Hillex II and Synthemax II microcarriers formed defined myotubes, whereas more defined myotubes could be observed of SMDCs previously cultured on Hillex II microcarriers. Also, AChE values of SMDCs previously culture on Hillex II microcarriers were higher than of SMDCs previously cultured on Synthemax II but also the SMDCs previously cultured on Synthemax II showed high AChE activity which was more than three times as high as the threshold ≥ 50 mUrel/200,000 cells. Thus, in summary it was concluded that both Hillex II and Synthemax II microcarriers are suitable for CD56$^+$ SMDC expansion.

**Example 23 - Effect of SMDCs seeding density on microcarriers in DASbox bioreactor system on AChE expression**

The impact of the seeding density on microcarriers in the DASbox bioreactor system on AChE expression following SMDCs differentiation was tested. As controls, cultivation in a spinner flask and 2D cultivation were included. Human SMDCs were obtained as described above after a 2-step digest (Example 20) and preliminary 2D culture (Example 21).

Conditions:

Cultivation in DASbox on 1200 cm$^2$ Hillex II at 90 rpm with 4 different seeding densities, namely, 600,000 cells, 2 mio cells, 3.4 mio cells and 6 mio cells

Control 1 (Standard): 2D cultivation in T75 flasks (AChE-activity tested after passage 1 (2D p1), passage 2 (2D p2) and passage 3 (2D p3)

Control 2 (3D Spinner flask): Cultivation in 500ml spinner flask on 1200 cm$^2$ Hillex with 3.4 mio cells seeding density.

Results are shown in Fig. 10 and the following table (Table 7):

| Condition | Seeding density [cells/cm$^2$] | Cultivation duration [days] | Cells harvested [in mio] | Doubling time [days] | % CD56$^+$ |
|---|---|---|---|---|---|
| 600,000 on Hillex II in DASbox | 500 | 10 | 57.6 | 1.5 | 99.7 |
| 2 mio on Hillex II in DASbox | 1666.7 | 6 | 35.4 | 1.5 | 99.4 |
| 3.4 mio on Hillex II in DASbox | 2833.3 | 6 | 70.3 | 1.4 | 98.8 |
| 6 mio on Hillex II in DASbox | 5000 | 4 | 49.5 | 1.3 | 99.2 |
| Standard 2D culture | n.a. | 10 | n.a. | Mean 1.1 | 96.0 |
| 3.4 mio in Spinner flask | 2833.3 | 5 | 46.8 | 1.3 | 99.0 |

No correlation between low seeding density and increased AChE expression in DASbox system was observed. The highest AChE value was observed in spinner flask condition. Moreover, a decrease of AChE activity in standard 2D culture over time (after each passage) was observed. As shown in Fig. 10, AChE activity of all 3D cultures (600,000 on Hillex II in DASbox, 2 mio on Hillex II in DASbox, 3.4 mio on Hillex II in DASbox, 6 mio on Hillex II in DASbox and 3.4 mio in Spinner flask) was higher than AChE activity of the third passage obtained in 2D. Under the tested conditions, three passages are at least necessary in 2D culture to obtain a comparable number of CD56 expressing cells as obtained in 3D culture. In addition, standard 2D culture without microcarriers results in a decreased proportion of CD56 expressing cells.

**Example 24 - Comparison of expansion of SMDCs in 2D and 3D**

The novel 3D expansion process according to the present invention was compared to the conventional adherent monolayer method (termed two-dimensional, or 2D).

Skeletal muscle (*musculus semitendinosus*) biopsy samples from three distinct human probands (samples designated as SMDC231228-1, SMDC231228-2 and SMDC231228-3) were processed by carefully excising fat, connective tissue, and tendons. The remaining pure muscle tissue was then finely cut into small fragments of approximately 1 mm$^3$. The processed samples were cooled as described in Example 10.

Afterwards, the processed and cooled sample for the 2D culture was digested by a one step digestion as described in Example 10. The subsequent primary culture phase was performed on uncoated cell culture flasks as also described in Example 10. Following primary culture, the cells underwent an 2D expansion method, involving three passages in T175 flasks (14 in total) and 1-stack CellSTACK® culture chambers (5 in total, by Corning, further termed "cellstacks").

In contrast, the processed and cooled sample for the 3D culture was digested by the 2- step digestion as described in Example 20. The subsequent primary culture phase was performed on culture vessels coated with Laminin 521 (Biolamina) as described in Example 21. Following primary culture, SMDCs were enzymatically detached using TrypLE™ Select Enzyme (1X, Gibco) and seeded into the DASbox® bioreactor system comprising *Hillex II* microcarriers. A maximum of 3.5 million cells were introduced into the bioreactor containing 1200 cm$^2$ of *Hillex II* microcarriers. Automated cell expansion was initiated at 37°C, wherein the medium volume within the reactor was 250 ml and the gassing (headspace) was 0.6 sL/min with 5%CO$_2$. Stirring was performed counterclockwise as follows: 90 min at 20 rpm for attachments, afterwards at 90 rpm with intervals of 1 min at 140 rpm after medium changes. Medium change (each 40 ml) was performed as follows: every 24 h from day 1 to 2, every 12 h from day 3 to 4, every 8h from day 5 on. Once the cell population was expected to have reached the target amount of 50 ± 10 million, manual harvesting was performed. For cell harvest, the bioreactor was disconnected from the DASbox® system, and cells were detached from the microcarriers using TrypLE™ Select as described in Example 2. The resulting cell suspension was filtered sequentially through 70 μm and 15 μm strainers to efficiently remove the microcarriers and obtain a purified single-cell suspension.

Cell count was monitored at every splitting step, and cell quality was evaluated through flow cytometry, immunofluorescence staining, myoblast fusion capability and AChE potency assays. Glucose and lactate levels were measured every 24 hours in the bioreactor, and representative measurements were done during one passage of standard 2D culture for comparison. Flow cytometry to assess viability and expression of CD56 and CD90 as well as AChE potency assay to determine fusion capability was performed as described in Examples 5 and 7. For additional information, expression of CD49f, CD105 and SSEA4 was assessed with flow cytometry using 5 μl CD49f-PE and CD105-PE antibody and 40 μl SSEA4-PE antibody.

Immunofluorescence staining was performed for Integrin alpha-7 (ITGa7), Myogenic factor 5 (Myf5), Desmin, myosin heavy chain (MHC), and smooth muscle actin (SMA) after expansion to 50 ± 10 million SMDC. SMDCs were seeded onto gelatine-coated 24-well plates. For MHC staining on differentiated cells, SMDCs were seeded onto gelatine-coated 24-well plates and allowed to differentiate for five to seven days. Afterwards cells were washed with PBS, followed by fixation with Paraformaldehyde (PFA). Cells were permeabilized by further washing steps with PBS containing 0.1% Triton (PBS-T). Following fixation, permeabilization and blocking (PBS-T with 3% Goat Serum), cells were incubated with primary antibodies at 4°C for 24 hours, followed by a 1-hour incubation with secondary antibodies at 37°C. Imaging and analysis were performed using the Incucyte S3 system (Sartorius AG).

The timing of aSMDCs harvest from microcarriers in the bioreactor was determined by estimating the confluency of cells attached to *Hillex II* microcarriers through visual inspection. To monitor cell growth, samples were taken from the bioreactor every 24 hours, and cell nuclei were stained with Syto24 to facilitate microscopic visualization. Harvest decisions were based on experience from previous cultivation processes.

## Results

The expansion of SMDCs on microcarriers within the DASbox® bioreactor system demonstrated a robust reliability, achieving cell yields of 50 ± 10 million cells. Both glucose and lactate levels remained within comparable ranges across both cultivation methods, without exceeding critical thresholds that could negatively impact cell culture.

Table 8: Comparison of SMDCs growth between the DASbox® bioreactor system and the standard 2D method.

| | | 3D | 2D |
|---|---|---|---|
| Duration of primary culture [days] | | 8.3 | 10.3 |
| Harvest from primary culture [mio cells] | | 6.1 | 1.9 |
| Seeded for further cultivation [mio cells] | | 5.5 | 1.9 |
| Overall cultivation duration [days] | | 16.3 | 19.7 |
| Mean doubling time during expansion [days] | | 1.9 | 1.9 |
| Harvest after expansion [mio cells] | | 48.2 | 53.8 |
| CD56% at harvest [%] | | 98.5 | 95.1 |
| CD105% at harvest [%] | | 98.2 | 99.6 |
| CD90% at harvest [%] | | 92.5 | 95.3 |
| SSEA4% at harvest [%] | | 77.0 | 77.4 |
| CD49f% at harvest [%] | | 98.5 | 81.2 |
| AChE activity [mUrel] | | 163.7 | 105.7 |
| Total medium consumption [1] | | 1.0 | 1.2 |

The observed doubling times of about 1.9 days, are within the range of previously recorded data for DASbox®

cultivations and are comparable to the doubling times of the standard 2D cultures. The reason for the reduced overall cultivation time of about 2 days in the DASbox® bioreactor system could be the application of Laminin 521 coating during the primary culture phase, which enhances cell proliferation efficiency and increases overall cell output.

Immunofluorescence staining of Integrin alpha-7 (ITGa7), Myogenic factor 5 (Myf5), and Desmin following SMDCs expansion revealed no significant differences between standard 2D cultivation and cultivation in the DASbox® bioreactor system. Staining for ITGa7 and Myf5 was consistently positive across all conditions. The percentage of Desmin+ cells exhibited variability depending on the biopsy material and cultivation method with no clear preference for either method. However, in all samples desmin expressing SMDCs were detected. Immunofluorescence staining for myosin heavy chain (MHC) and smooth muscle actin (SMA), markers of prematurely fused myoblasts and smooth muscle cells or myofibroblasts, respectively, demonstrated no detectable signal under neither condition, as expected.

Interestingly, an increased CD49f expression after cultivation in DASbox® bioreactor system was observed. In contrast to other surface markers, CD49f expression exhibited a notable shift between the different cultivation methods. During standard 2D cultivation, CD49f expression was stable to decreased (-27%). However, following cultivation in the DASbox® bioreactor system, CD49f expression markedly increased, with expression increasing by 3 to 10%, resulting in 98 to 99% of cells being CD49f +. The dynamic conditions within the stirred DASbox® bioreactor system may promote increased cell adhesion to the microcarriers, potentially driving the observed upregulation of CD49f. This heightened CD49f expression might also contribute to enhanced fusion potential, as evidenced by the higher acetylcholinesterase (AChE) activity in bioreactor-cultured cells.

In fact, an enhanced myotube formation following cultivation in the DASbox® bioreactor system was observed. SMDCs expanded under both standard 2D conditions and in the DASbox® bioreactor system demonstrated the ability to form multinucleated myotubes, each containing at least three nuclei. These cells also exhibited relative acetylcholinesterase (AChE) activity exceeding 50 mUrel/200,000 cells. Notably, cells obtained from 3D cultivation showed enhanced AChE activity in comparison to cells obtained from 2D cultivation.

Thus, it was shown that 3D cultivation of SMDCs on microcarriers achieves comparable cell yields (50 ± 10 million cells) to 2D protocols of cultivation but i) with a reduced overall cultivation time and medium consumption, starting from biopsy digestion, ii) without the need for passaging during the expansion phase until harvest within the bioreactor, and (iii) with an resulting expanded population of SMDCs showing increased CD56 and CD49f + expression and enhanced myotube formation and AChE activity.

The results demonstrate that SMDCs isolated and expanded using the method according to the present invention is successful for providing an expanded SMDCs population which is able to form myotubes which can regenerate skeletal muscles. A comparison with the parallel 2D culture method revealed high comparability between both processes with observed benefits for cell growth and fusion capability in the 3D system. Thus, the 3D system is a promising solution for semi-automated expansion of SMDCs, enhancing the efficiency and consistency of the cultivation process of SMDCs.

## Claims

1.  A method for obtaining an expanded population of skeletal muscle derived cells (SMDCs), the method comprises the following steps:

    (a) providing a suspension in a container comprising

    (i) skeletal muscle derived cells (SMDCs) obtained from skeletal muscle tissue,
    (ii) microcarriers, and
    (iii) culture medium,

    (b) cultivating the suspension under

    mixing in a given volume, and
    changing the medium several times, and
    conditions allowing the expansion of said SMDCs,

    thereby obtaining an expanded population of SMDCs comprising at least 60% CD56 expressing SMDCs,
    (c) optionally harvesting the expanded population of SMDCs attached to the microcarriers.

2.  The method according to claim 1, wherein the microcarrier density in step (b) remains constant, preferably in the range of about 2 to about 8 cm$^2$/mL.

3. The method according to claim 1 or 2, wherein step (b) is performed until a cell number of about 40 million cells to about 200 million cells or a cell density of about 1.6 x $10^5$ cells/ml to about 8 x $10^5$ cells/ml is obtained.

4. The method according to any one of claims 1 to 3, wherein the cells in step (a) comprise at least 30% CD56 expressing SMDCs, more preferably at least 40%, 50%, 60%, 70%, 80%, 90%, 95% or 98% CD56 expressing SMDCs and/or wherein the population of expanded SMDCs obtained in step (b) comprise at least 60% CD56 expressing SMDCs, more preferably at least 70%, 80%, 90%, 95% or 98% CD56 expressing SMDCs.

5. The method according to any one of claims 1 to 4, wherein the proportion of CD56 expressing SMDCs in the total cell number increases during expansion or decreases at most by 10%.

6. The method according to any one of claims 1 to 5, wherein the cultivation in step (b) results in that the proportion of CD49f expressing cells in the total cell number of SMDCs increases during expansion and/or wherein at least 80%, more preferably at least 90%, of the expanded population of SMDCs obtained in step (b) express CD49f.

7. The method according to any one of claims 1 to 6, wherein the SMDCs in step (a) have been preferably obtained by a method comprising the following steps:

(i) cooling of a sample obtained from skeletal muscle tissue in a liquid,
(ii) processing of the sample and cooling the processed sample, preferably at a temperature of about 1 to about 16°C for about 2 to about 60 hours,
(iii) resuspending the processed sample of step (ii) in a mixture comprising one or more enzymes under conditions allowing an enzymatic digestion of the processed sample by said one or more enzyme, thereby obtaining a suspension comprising single cells and remaining tissue fragments.

8. The method according to claim 7, wherein step (iii) comprises a first incubation step using a first enzyme and a second incubation step using a second enzyme.

9. The method according to claim 8, wherein the first enzyme is a protease, more preferably an endopeptidase such as collagenase and the second enzyme is a distinct protease or endopeptidase such as Trypsin or a recombinant protease or endopeptidase such as a recombinant Trypsin alternative or animal-component-free trypsin substitute.

10. The method according to any one of claims 1 to 9, wherein the SMDCs provided in step (a) have prior to step (a) be cultured by a method comprising the following steps:

(I) plating skeletal muscle derived cells (SMDCs) obtained from skeletal muscle tissue onto a dish and incubating the dish under conditions allowing cells including CD56 expressing SMDCs to adhere to the surface of the dish, wherein the dish is preferably coated with Laminin, a recombinant Laminin, Laminin 521 or Laminin 511
(II) propagating the adherent cells including CD56 expressing SMDCs obtained in step (I), and
(III) detaching the cells including CD56 expressing SMDCs obtained in step (II) from the surface of the dish.

11. The method according to any one of claims 7 to 10, wherein the steps of claim 10 are performed after step (iii) and the suspension of single cells obtained in step (iii) is plated onto the dish in step (I).

12. An expanded population of SMDCs obtained by the method according to any one of the preceding claims.

13. An expanded population of at least 40 million SMDCs comprising at least 60% CD56 positive, at least 60% CD105 positive and at least 60% CD90 positive cells and at least 80% or 90%, 95% CD49f positive cells.

14. An expanded population SMDCs according to claim 12 or 13 for use in a method for prevention and/or treatment of the human or animal body by surgery or therapy or for use as a pharmaceutical composition.

15. An expanded population of SMDCs according to claim 12 or 13 for use in a method of preventing and/or treating neuromyopathies, myopathies and/or muscle dysfunctions, in particular wherein the neuromyopathies and/or myopathies is an incontinence such as fecal incontinence and/or urinary incontinence and/or wherein the muscle dysfunction is a skeletal muscle dysfunction such as incontinence, preferably fecal incontinence and/or urinary incontinence.

**Fig. 1**

**Fig. 2**

A

>10 cm²/ml        <10 cm²/ml

6 days cultivation

B

Fig. 3

EP 4 763 976 A1

34

Fig. 4

**Fig. 5**

A

| Biopsy | Total days of differentiation | AChE activity [mUrel/ml]* |
|---|---|---|
| SMDC230131-1 | 3 | 211 |
| SMDC230131-2 | 3 | 286 |
| SMDC230131-3 | 3 | 308 |

\* Threshold of ≥ 50 mUrel/ml required to pass test

**Fig. 6**

| Batch 1.2 | Batch 2 |
|---|---|

Tube formation (A)

| AChE activity [mUrel] (B) | 50 | 77 |

| Batch 3 |
|---|

108

Fig. 7

2-step digest

1-step digest

Before passing cell strainers

After passing cell strainers

**Fig. 8**

A

B

**Fig. 9**

**Fig.10**

**Fig. 11**

**Fig. 11**

Europäisches
Patentamt

European
Patent Office

Office européen
des brevets

# EUROPEAN SEARCH REPORT

**Application Number**

EP 24 22 3011

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANDREASSEN R. CHRISTEL ET AL: "Production of food-grade microcarriers based on by-products from the food industry to facilitate the expansion of bovine skeletal muscle satellite cells for cultured meat production", BIOMATERIALS, vol. 286, 1 July 2022 (2022-07-01), page 121602, XP093278078, AMSTERDAM, NL ISSN: 0142-9612, DOI: 10.1016/j.biomaterials.2022.121602 * abstract * * page 3 * * figures 3-5, S4 * | 1,5-12 | INV. C12N5/077 ADD. A61K35/34 |
| X | CARTAXO ANA LU?SA ET AL: "Developing a Cell-Microcarrier Tissue-Engineered Product for Muscle Repair Using a Bioreactor System", TISSUE ENGINEERING. PART C, METHODS DEC 2008, vol. 29, no. 12, 1 December 2023 (2023-12-01), pages 583-595, XP093278077, US ISSN: 1937-3384, DOI: 10.1089/ten.tec.2023.0122 * abstract * * figures 1, 6, 8 * | 12,14,15 | |
| | | | TECHNICAL FIELDS SEARCHED (IPC) |
| X | WO 2019/115790 A1 (INNOVACELL BIOTECHNOLOGIE AG [AT]) 20 June 2019 (2019-06-20) * claims 1, 11, 15 * | 12,14,15 | C12N A61K |
| | -/-- | | |

The present search report has been drawn up for all claims

| Place of search | Date of completion of the search | Examiner |
|---|---|---|
| Munich | 16 May 2025 | Erener Caner, Süheda |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | **Application Number** EP 24 22 3011 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NORRIS SAM C.P. ET AL: "Emulsion-templated microparticles with tunable stiffness and topology: Applications as edible microcarriers for cultured meat", BIOMATERIALS, vol. 287, 1 August 2022 (2022-08-01), page 121669, XP093278056, AMSTERDAM, NL ISSN: 0142-9612, DOI: 10.1016/j.biomaterials.2022.121669 * abstract * * page 3, last paragraph * ----- | 1,5-12 | |
| X | WO 2024/115761 A1 (UNIV ZUERICH [CH]) 6 June 2024 (2024-06-06) * claim 1 * * page 19, line 29 - line 34 * * claim 12 * ----- | 1-3, 5-12,14, 15 | |
| X | ANDREA FRUDINGER ET AL: "Skeletal muscle-derived cell implantation for the treatment of sphincter-related faecal incontinence", STEM CELL RESEARCH & THERAPY, vol. 9, no. 1, 13 September 2018 (2018-09-13), XP055599139, DOI: 10.1186/s13287-018-0978-y * abstract * * page 3, last paragraph * * page 4, paragraph 1 * * figures 3, 6 * ----- -/-- | 12-15 | TECHNICAL FIELDS SEARCHED (IPC) |

The present search report has been drawn up for all claims

| Place of search | Date of completion of the search | Examiner |
|---|---|---|
| Munich | 16 May 2025 | Erener Caner, Süheda |

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TZIMOROTAS DIMITRIOS ET AL: "Expansion of bovine skeletal muscle stem cells from spinner flasks to benchtop stirred-tank bioreactors for up to 38 days", FRONTIERS IN NUTRITION, vol. 10, 7 August 2023 (2023-08-07), XP093278079, ISSN: 2296-861X, DOI: 10.3389/fnut.2023.1192365 * abstract * * figures 1, 3, 4 * * page 8, paragraph 1 * * 2.2; page 2 * * 2.4; page 3 * * page 5B * ----- | 1,3-12 | |
| | | | TECHNICAL FIELDS SEARCHED (IPC) |

The present search report has been drawn up for all claims

| Place of search | Date of completion of the search | Examiner |
|---|---|---|
| Munich | 16 May 2025 | Erener Caner, Süheda |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 3011

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2025

| Patent document cited in search report | Publication date | Patent family member(s) | Publication date |
|---|---|---|---|
| WO 2019115790 A1 | 20-06-2019 | AU 2018383015 A1 | 28-05-2020 |
| | | BR 112020011853 A2 | 24-11-2020 |
| | | CA 3085483 A1 | 20-06-2019 |
| | | CL 2020001480 A1 | 30-10-2020 |
| | | CN 111448307 A | 24-07-2020 |
| | | CY 1126049 T1 | 15-11-2023 |
| | | DK 3724319 T3 | 01-05-2023 |
| | | EP 3724319 A1 | 21-10-2020 |
| | | EP 4223872 A2 | 09-08-2023 |
| | | ES 2948712 T3 | 18-09-2023 |
| | | FI 3724319 T3 | 04-05-2023 |
| | | HR P20230474 T1 | 21-07-2023 |
| | | HU E062005 T2 | 28-09-2023 |
| | | IL 275288 A | 30-07-2020 |
| | | IL 309687 A | 01-02-2024 |
| | | JP 7361398 B2 | 16-10-2023 |
| | | JP 7494430 B2 | 04-06-2024 |
| | | JP 2021506285 A | 22-02-2021 |
| | | JP 2023085507 A | 20-06-2023 |
| | | KR 20200099173 A | 21-08-2020 |
| | | KR 20250026373 A | 25-02-2025 |
| | | LT 3724319 T | 12-06-2023 |
| | | PL 3724319 T3 | 24-07-2023 |
| | | PT 3724319 T | 07-06-2023 |
| | | RS 64241 B1 | 30-06-2023 |
| | | RU 2020120580 A | 14-01-2022 |
| | | SI 3724319 T1 | 31-07-2023 |
| | | SM T202300147 T1 | 20-07-2023 |
| | | US 2021069255 A1 | 11-03-2021 |
| | | US 2023285467 A1 | 14-09-2023 |
| | | WO 2019115790 A1 | 20-06-2019 |
| | | ZA 202002410 B | 29-09-2021 |
| WO 2024115761 A1 | 06-06-2024 | NONE | |

EPO FORM P0459

For more details about this annex : see Official Journal of the European Patent Office, No. 12/82

## REFERENCES CITED IN THE DESCRIPTION

**Patent documents cited in the description**

- WO 2019115790 A
- WO 2023012334 A
- WO 2024115761 A1

**Non-patent literature cited in the description**

- **THURNER et al.** *PLoS ONE*, 2018, vol. 13 (3), e0194561
- **WILSCHUT et al.** *Stem Cell Res*, 2011, vol. 7, 112-123